# EUROPEAN PATENT APPLICATION

(11) **EP 3 981 878 A1**
(43) Date of publication of application: **13.04.2022**
(21) Application number: 20823252.0
(22) Date of filing: 09.06.2020
(51) Int. Cl.: C12N 15/115, A61K 31/7115

(54) **FPR2 RECEPTOR AGONIST APTAMERS AND USES THEREOF**

(30) Priority: 10.06.2019 ES 201930524
(71) Applicant: Universidad Carlos III de Madrid, 28919 Leganés - Madrid (ES); Centro de Investigaciones Energéticas, Medioambientales y Tecnológicas, O.A., M.P., 28040 Madrid (ES); Fundación para la Investigación Biomédica del Hospital Universitario Ramón y Cajal, 28034 Madrid (ES); Aptus Biotech, S. L., 28035 Madrid (ES); Fundacion Instituto De Investigacion Sanitaria Fundacion Jimenez Diaz, 28040 Madrid (ES)
(72) Inventor: CARRETERO TRILLO, Marta, 28040 Madrid (ES); DE ARRIBA PÉREZ, María del Carmen, 28919 Leganés (Madrid) (ES); DEL RÍO NECHAEVSKY, Marcela Andrea, 28919 Leganés (Madrid) (ES); FERNÁNDEZ GÓMEZ-CHACÓN, Gerónimo, 28035 Madrid (ES); GONZÁLEZ MUÑOZ, Víctor Manuel, 28034 Madrid (ES); CARRIÓN MARCHANTE, Rebeca, 28034 Madrid (ES); MARTÍN PALMA, Elena, 28034 Madrid (ES)
(74) Representative: Pons
(86) International application number: PCT/ES2020/070378
(87) International publication number: WO 2020/249841

(57) **Abstract**

The present invention is related to FPR2 receptor agonist aptamers and uses thereof. The present invention is related to a nucleic acid aptamer that is able to specifically bind to the FPR2 receptor and activate said FPR2 receptor, comprising a nucleotide sequence with a sequence identity of at least 70% with the sequence SEQ ID NO. 1, SEQ ID NO. 2 or SEQ ID NO. 3.

SEQUENCE LISTING

<110>  Universidad Carlos III de Madrid
       Centro de Investigaciones Energéticas, Medioambientales y
       Tecnológicas, O.A., M.P.
       Fundación para la Investigación Biomédica del Hospital
       Universitario Ramón y Cajal
       Aptus Biotech, S.L.
       Fundación Instituto de Investigación Sanitaria Fundación
       Jiménez Díaz

<120>  FPR2 RECEPTOR AGONIST APTAMERS AND USES THEREOF

<130>  EP3473.7

<150>  ES P201930524
<151>  10-06-2019

<160>  10

<170>  PatentIn version 3.5

<210>  1
<211>  76
<212>  DNA
<213>  Artificial Sequence

<220>
<223>  ApFP3.5a Aptamer

<400>  1
gcggatgaag actggtgtgg gcgggggtct taggctgtac ggggctgttc aggtgcttgc      60

cctaaatacg agcaac                                                      76


<210>  2
<211>  76
<212>  DNA
<213>  Artificial Sequence

<220>
<223>  ApFP3.8a Aptamer

<400>  2
gcggatgaag actggtgtgg ggatcaggaa ctctgaaatg gcagtctatg tttcaatggc      60

cctaaatacg agcaac                                                      76


<210>  3
<211>  76
<212>  DNA
<213>  Artificial Sequence

<220>
<223>  ApFP4.5b Aptamer

<400>  3
gcggatgaag actggtgttg tggcgcttcg ggcctgtccc tttatatccg tagattgagc      60

cctaaatacg agcaac                                                      76

<210>  4
<211>  18501
<212>  DNA
<213>  Homo sapiens

<400>  4
gctttaatgg cattaggaca gtgggctggg cctgtttaga aactcatgta cctgactgta      60

tttctcagcc tcagtctctg tgggatgcag aggttcagtg ctctcaacaa caagggccaa     120

gaaatccaag tgtccgggcc cctgctccct ccaagggtca aggtaaagaa agcagcaagc     180

actgtggtga gaagaatcag agggaggcaa tgaaaggaag agaagaagtc atgagggatg     240

gagaccagaa acacagagag aggtaaggga gtgggggaag ttgagacagt tggcaggaca     300

gcgcgtccac acccatgcac accaccaaga ttaaccccat aatcctctga tcttgtggaa     360

gctcatttgt agtcttctag cccaaagaaa taaccgtttt tctgaaatgt taagcatcct     420

tccaagggta tcagagtgat atggagaaat atggtaagac agttacagaa tcagagataa     480

cgacactgat tctaaaagca ttattaccaa ggcattgttg cattaagact gttttttctt     540

ttcttttaga gacagtatcc tgctacgttg cccaggcatg tctcagaccc ctgggtttag     600

tgatcctcct gcctcagcct ccccaaatgc tgggattaca ggcatgagcc gggctgtttt     660

taattctaag taatttataa tttacaatta cacctaacaa tggcttcaac aaggggtgtc     720

caatcttttg ccttccctgg gccacactgg aagaagaaga attgtcttgg gccacacata     780

aaatacacta acactaacga tacctgatga gctaaaaaaa aaaaaaaaaa aaaaaatccc     840

ccaaaaatct gataatgttt taagaaagtt tatgaatttg tgttgggctg cattcaaagc     900

catcttaggc catgggctgg acaagcttag cttagaacca agacgaaatt tatggtttac     960

tttacaagaa ttttgcaaga gggtaatact aggttaggtt atatctggaa aatatcatca    1020

agaactacat tcattttaat cttttctctt ttttatccct aatatggtga ctttttttcc    1080

tggggattgc cacttcatgg ctccaagaaa gctaccacag ctcctgactt cacattacag    1140

caggggtccc caacctccag gccatagacc aatacacaga ccgataccgg tctgtggcct    1200

gttaggaagt gggctgcaca gcaggtgact ggcaggtgag caagagaagc ttcatctgta    1260

tttatagcca ctccccattg ctggcattac tgcctgagct ctgcctcctg tcagattagt    1320

ggcggcatta gattctcata acagtgtgaa ccctactgtg aactgtgcat gccagggatc    1380

tgggttgtgt gctccttatg agaatctaat gcctgatgat ctgtcactgt ctcccatcac    1440

tcccagatgg gactgtctag ttgcgggaaa gcaagctcag ggctcccacg gattctacat    1500

cattgtgagt tgtataatta tttcattgtg tattacaatg taataataat aatagaaata    1560

aagtctacaa taaatgcaat gtgcttaaat catcctgaaa ccatctaccc cgccacccca    1620

tggagaaatt gttttttatg aaactggtcc ctggtgccaa aaatgttggg gactgctaca    1680

ctccagcatc ccacaccagg aggatgggga tgagatggag aatcactgaa gatctttaat 1740
cagaagggtg atatgttcat ttgtacttta taaacatttg tgtttgtatt ttataaacat 1800
tttcttggga tcataacaac agatgcattt tagaagattc ccaaggtatg aagggtaaca 1860
aatgaagacg tgcctaggag tcacatggaa gccaacactt tgtaagggga ttaaaataaa 1920
gggaatggca gaggacatca ttatgcacag aggagaaggg cccaaatgtc aagagaaaac 1980
aagagacaag ggactccaca gagagtaatt gccagaaaaa tttgtaggtc ttttgaagtc 2040
tgactgttct tcctaaaacg ggggtcctgg gacaccccta cctgtttccc atgagccctc 2100
ttttctaaaa cataagtagg ttttcttcct ggctaccaaa tggcctgtga ttagcacact 2160
aggttgggca aaaatagagc caagtgtgtg ttccaaagtg actatggtga agaaaaaagc 2220
gaaagtctta aaatggactt ccacttctgt gtttgcgtaa ggctaaaagg gaagatgtct 2280
tagggttgac cggaatggga gtattcctgc ctatgctgca aaagtccctg actcttctgg 2340
taactgaaac atgagaaatg gcctcggcga gcagcacagc ctcccagtaa agcactggac 2400
tgaggtggta aagccagatt gcctggacta gaatcccagg actactgttc actagctccg 2460
gacaagccag ctaatccagg ggtctcaaac atttccccca actttttttt tttgagacag 2520
agtctcactg tgtcacccag gctggagtac ggtggtgtga tcttggctca ctgcagtttt 2580
gcctcctagg ttcaagtgat tcccctgcct cagcctctcc agtagctggg attacaggtg 2640
cccgccacca cgcctggcta atttttgtat tttttaagta gagatatggt tttgccacgt 2700
tgaccaggct ggtcttgaac tcctgacctc aaacaatcac ttgccttggc ctcccaaagt 2760
gctgggatta caggacggaa ccactgcact gggccacaaa catttcctat gaagagccag 2820
atggtcaata tatttcattt ggcaggccat agggtctcca tcacaactcc ttggctctgc 2880
tgttgtagcc tgaaaacagc cacaagcaaa gttgtaaatg aggagcatga ctgtatgtca 2940
ataaaacttt atttaaaaca cgacaattgg acttcatata agttttgcat ggcacaaaat 3000
acccttcatt tgattttctt tagccggtaa gaaatattaa agccattctt ggctctcagg 3060
ccatacaaaa acaagcggtg gctagatttg acccctgatt taactgcact gggtcccaga 3120
cacttcacct gtgaattgag agcaatgata cttacagagg tgttttctgg agcagaacaa 3180
ataagatcag tgatacaaag cacttggcat gaattccagt gcaataagta ttatcaatat 3240
cttagtaatt acataaacct ctctatctgc tgtttcacgt tctctgcggt gaacccatcc 3300
atgcttaaaa cttgaaccac acctcagcta acattcacca gacccctgtc tctagctcag 3360
acctgccctt gatgttcaga cacatcattt tgggttcctg tttcctttca aactcatctg 3420
cagcctttgc tctcctcacc ttctcccaca actctgttaa ggactaaatt gtgtcacatt 3480
ccccttcaaa ttcttaggtt gaagccctca ccccatgata tggtttggct gtgtcccaac 3540

cctgatctca tcttgaattg tagctcccat aattcccacg tgttgtggga ggaaccaggt 3600

gggagatagt tgtttcatgg ggacgatttc ccccataatg ttctcatggt agtgaatatg 3660

tctcacgaga tctgacgatt ttatgagggg tttccccttt cacttggttc tcattctctt 3720

ttgcctgctg ccatgtaaga tgtccctttg ttcttccttc gccttccgca atgattgcga 3780

ggcctcccca gccacatgga actgtgagtc cattaaacct cttcccttta taaattaccc 3840

agtcttgggt atgtcattat tagcagcatg agaattgacc aatacacccc agcatgtcag 3900

aatgtgactg tatttgggga tacaaccttt aaagaggtaa ttagctgaaa ctgaggtcat 3960

tagggtgggc cccaactcaa tatgactgat atccttatga aaagaggaga tccagacaca 4020

gacaggcata gagggaaaac tgtgtgaaaa catgaggagg aggcggtatc tgcaagccaa 4080

ggagagagga ctcaggagga atcaaccctg ctgacacctt gatcttaaac ttccagctga 4140

taaaactgtg agaaaatata tttctattgt tgtagccacc cagccaatgg tattttgtta 4200

tggcagctct aacaaactaa acactctgct tcttttcatt gactctcgtc tcaatgacag 4260

cccccaaatc cacgcagtta ccttggtgca acagttctga agaaaatggc cacaatattc 4320

ttggacactc cttccactga caggaaggca ggcatattgt atcgcctctc ccctggaacc 4380

tgggtagggt tgggactact ttgaccaaag aacgtggtag aaatgacttc cacgtagctt 4440

tcaaggctag gtcaaaaaag gctaggaagc atcttctggt tctcctggga cacgtgttct 4500

ggaggaaggc agccattgtc ctgggacttt gattcctctg agaccaccat gctggaaagg 4560

ctgcctgtag gcacttggtg caataccagc ctagttcagc atttgagtca ttccaaccca 4620

ggcatctgac atgtgaatga aggagctcta gatgactcca attcacagtc ttctagtcac 4680

cccccagacc tttgagtttt gggtggcaga gacaagcatc tctgtgcctt atcctaattc 4740

ccaacccaca taatccatga acgtaatgaa atggttgttt tacatgctat gatttgggtg 4800

gttatacagc aattatgaat tatgaatgtg ggcttcataa agacccttca tgcttcctca 4860

ttctccagag ccatttggtc tccaagctca gtctaccaag cctggaaatc tctccctaaa 4920

ctacacctcc tctctagcac actactccag cacaggcaat tattcgacaa gttcaccttg 4980

atctctggca acttcttacc ctttaagatc tctgcctcca actgggagca ttctcagagc 5040

catccctttg cagggggact ctagagcaat gttttctgga agcacacacc tgacaagtca 5100

ctctcctgct taaatagctt ttaagcagaa gaaattgtaa gaaagaaatt tttttttaga 5160

ccaatatcct tgatgaacat tgctgcaaaa atcctcaata aaatactgac aaaccaaatc 5220

cagcagcaca tcaaaaagct tatccaccac gaccaagtgg gcttcatccc tgggatgcaa 5280

ggctggttca atatacgcaa atcaataaat gtaatccagc atataaacag aaccaaagac 5340

aaaaaccaca tgattatctc aatagatgca gaaaaggcct ttgacaaaat tcaacaaccc 5400

ttcatgctaa aaactctcaa taaattgggt attgatggga cgtatctcaa aataataaga 5460

gctatctatg acaaacccac agccaatatc atactgaatg ggcaaaaact agaagcattc 5520

cctttgaaaa ctggcacaag acagggatgt cctctctcac cactcctatt caacatagtg 5580

ttagaagttc tggccagggc aattaggcag gagaaggaaa taaagggtat tcaattagga 5640

aaagaggaag tcaaattgtc cctgtctgca gatgacatga tggtatatct agaaaacccc 5700

attgtctcag cccaaaatct ccttaagctg ataagcaact tcagcaaagt ctcaggatac 5760

aaaatcaatg tacaaaaatc acaagcattc ttatacacca ataacagaca aacagagagc 5820

caaatcatga gtgaactctc attcacaatt gcttcaaaga gaataaaata cttaggaatc 5880

caacttacaa gggacgtgaa ggacctcttc aaggagaact acaaaccact gctcaatgaa 5940

ataaaagagg acacaaacaa atggaagaac attccatgct catgggtagg aagaatcaat 6000

atcgtgaaaa tggccatact gcccaaggta atttacagat tcactgccat ccccatcaag 6060

ctaccaatga ctttcttcac agaattggaa aaaactactt taaagttcat atggaaccaa 6120

aaaagagccc gcattgccaa gtcaatccta agccaaaaga acaaagctgg aggcatcacg 6180

ctacctgact tcaaactata ctacaaggct acagtaacca aaacagcatg gtactggtac 6240

caaaacagag atatagatca atggaacaga acagagccct cagaaataac gccgcatatc 6300

tacaactatc tgatctttga caaacctgag aaaaacaagc aatggggaaa ggattcccta 6360

tttaataaat ggtgctggga aaactggcta gccatatgta gaaagctgaa actggatccc 6420

ttccttacac cttatacaaa aattaattca agatggatta aagacttaaa tgttagacct 6480

aaaaccataa aaaccctaga agaaaaccta ggcattacca ttcaggacat aggcatgggc 6540

aaggacttca tgtctaaaac accaaaagca atggcaacaa aagccaaaat tgacaaatgg 6600

gatctaatta aactaaagag cttctgcaca gcaaaagaaa ctaccatcag agtgaacagg 6660

caacctacaa aatgggaaaa agttttcaca acctactcat ctgacaaacg gctaatatcc 6720

agaatctaca atgaactcaa acaaatttac aagaaaaaaa caaaaaactc tatcaaaaag 6780

tgggcaaagg atatgaacag acacttctca aaagaagaca tttatgcagc caaaagacac 6840

atgaaaaaat gctcatcatc actggccatc agagaaatgc aaatcaaaac cacaatgaga 6900

taccatctca caccagttag aatggcaatc attaaaaagt caggaaaaaa caggtgctgg 6960

aaagggagaa aataggaaca cttttacact gttggtggga ctgtaaacta gttcaaccat 7020

tgtggaagtc agtgtggtga ttcctcaggg atctagaact agaaatacca tttgacccag 7080

ccatcccatt actgggtata tacccaaagg actataaatc atgctgctgt aaagacacat 7140

gcacacgtat gtttattgca gcactattca caatagcaaa gacttggaac caacccaaat 7200

gtccaacaat gatagactgg attaagaaaa tgtggcacat atacaccatg gaatactatg 7260

cagccataaa aaatgatgag ttcatgtcct ttgtagggac atggatgaaa ttggaaatca 7320

tcattctcag taaactatca caaggacaaa aaaccaaaca ccgcatgttc tcactcatag 7380

atgggaattg aacaatgagg aaacatggac acaggaaggg gaacatcaca ctctggggac 7440

tgttgtgggg tgggggaggg ggggagggat agcattggga gatataccta atgctaaatg 7500

acgagttaat gggtgcagca caccagcatg gcacatgtat acatatgtaa ctaacctgca 7560

cattgtgcac atgtacccta aaacttaaag tataataata aaaatatata tatatataag 7620

aaagaaattt taagaagaaa ttctaagttc tttttacagc attcaagacc aaccaagaaa 7680

ttgcctctgc cgccctctac atacttaatg cttattgctg tctgccttat catctatgct 7740

ctggtaaaca aaagtaacct gcttttttgt gacccctttc gtgggtattt ttactcccct 7800

ctgacaagaa tgcattattt tttctccttt atctgagtct ttaagactca gcctacatgt 7860

tccctcctcc ggatattgac tctagatccg tgaatctgag ttagtggttc cttttagagg 7920

acctcacaag gagccaggca tctgtctatc actacgtgcc cccaccctat tgtaactaag 7980

cactgcattc tcacctctct attcaggtgg tccgcagagc ccatgtctga ttgatctcta 8040

tgtctccagc agccagcaag gaagcacctc tttagagacc tgcacctata caatacctac 8100

caccttttat ttctcgatat gtgaactcca ttgagaacaa acgagtaaat gtaggtaatg 8160

tgccttcttc ttttcttttc ttttcttttt ttttttagat ggagtctcgc tctgttgccc 8220

aggctggagt ttagtggcac aatctcggct cactgtaacc tccgcctccc aggttcaagc 8280

gattctgccg cctcagccta ccaagtagtt gggattacag gtgcccacca ccacgcccag 8340

ctaatttttt ttgtattttt agtagaggtg gggtttcatg atgttggcta ggatggtttt 8400

caactcctga cctcaagtga tccacccacc tcggcctccc aaagtgctag gattacaggt 8460

gtgagccaca gcgcccagcc agtaatgtgc cttcttaagt tctgtgagcc attctaacaa 8520

attatcagaa cagaggaagg ggttataaac atccccccac ccccgattta tagccagtca 8580

gtcagaagta caggtggcca cctgggactt ggattggtgt ctgaagtgag gacagttttg 8640

ggagagtgag ccctttaact tgtgggatct gacactaact ccaggtagac agcgtcggag 8700

ctgaattgaa ttgtgagata cccagtggtg tccccagaga actggagaat tgcttgatat 8760

ggaaaagacc cacacatttg atgccagaag tactgcataa gtcgagaatt gagtttgact 8820

taatcatcat atttgggctt gattgcgtgg ctgaaactct tcccacttca gtaattgttt 8880

ctttcatttt catgaaactc tgaagaagga agggctggac attcagattc cttgaccctt 8940

gacatttgga agcatgaact ccagtctctc acagaaggct agaggtgaag gaacattcag 9000

acacattggt ttctaagaag agtccgctga caacataccc aaggtgtctt ctgaaattta 9060

taagaaatcc tgagtttctg ttaggggatt ggctccagct ccattgtccc tcccccatca 9120

ttcagtagtc tccgcgaaag cccttagagc cggtgttgct ccacaggaag ccaagaagca 9180

cacaggaaaa ggagcttagc tgctggtaag ttggggactg acaatttaaa atttatcaat 9240

ggaactcctg ctctctgaca aacacctcct aaattctaga gcagtgctgt aatggagcat 9300

ccacttgcca caggtggctt ttgagtgcct atgtttctat cttgatctga aatgtgctta 9360

agtgtaaaat atacttagtg tagtttaatc attagtatga tcaaaataat acaatttatc 9420

tcgttaataa ctttcatttt ggtaacgtgt tccaatgatt tttttatttt gcggggttaa 9480

atgaaacata ttattataat taatttctcc caatgatttt acttttttaa catggctact 9540

agaaagttgt aagtcacgtg tgtggctggc atttagggct tgcattaccc tgtctctact 9600

aaaaatgcaa acaacaaact agccaggcgt agtggtggga gcctgtaatc ccagctactc 9660

aggaggctga ggcaggagaa tcgcttgaac ctaggaggcc gaggttgcag tgagccgata 9720

tcgtgccatt gcactccagc ctgggcaaga agagtgaaac tctaactcaa agataaataa 9780

acatgtgaag gagccaagtg tgaggagctt aagaggaaga gcatggtagg cagaaggaac 9840

agagaaggca gaagccccga gatgacaatg agctgcatgt tcaaggaatc agagcccgtt 9900

atgggtggag cccagtgtgt cccacgagag tgcagagatg aggtctgagg aatgcacagg 9960

gcaggtccag gggtacttga gggaacttaa gataaggata agttagaagt ttggaagcca 10020

ttagaaggtg ttgaaaatag catcaccatt ttattggacc tttttttttt ttttttttga 10080

gacagaatct tgctctgtcg cccaggctga agtgcagtgg cgcagtctcg gctcactgca 10140

aactctgcct ctcagggtca agcgattctc ctgcctcagc ctcccaagta gctgggatta 10200

caggcacccg ccatcacgcc cagctaattt ttgtgttttt ttagtagaga cggggtttca 10260

tcatgttggg caggttggtc ttgaactcct gacctcaggt gatccaccca cctcagcctc 10320

ccaaagtgct gggattacag gcatgagcca ctcccggccc ttattggact tttttttttt 10380

ttttgagaca gagttttgct cttgttgccc aggctggagt gcaatggcac gatctcagct 10440

cactgcaacc tccgcctccc tggttcaagc gattctcctg cgtcagcctc ccgagcatct 10500

ggaattacag gcacgtgcca ccacgcccag ctaattttgt atttttagta gagatggggt 10560

ttcttcatgt tggccaggtt ggtctcgaac tcctgacctc tggtgaccca cctgcctcgg 10620

cctcccaaag tgctgggatt acaggtgtga gccactgcac ctggccctta ttgaactttt 10680

taaaagctca tttggtttcc ttctggagat tggattgtag ggcaggggaa agtaggacca 10740

ggaacaacct atttgcaaag ttggcgcaaa cattcctgcc tgacaggacc atggacacag 10800

gttgtagaga tagagatggc tctggctgtg cattcagcag attctgtaga tagaattaat 10860

aggacttgga tgggattgtg gtgagagaaa gtgaaatgaa agataagttc tagtttggaa 10920

gttttaacaa ctgaatgttt aaactcaaat agacacaaaa tattggaaga gtggcaggtt 10980

tgggaggatg agacaatcaa ctgtttggtt gagccacgtt aggtttgaaa tgtctacggg 11040

actcccgtgg ggagaggtta tatcagactg gagcaccaga gagaggccaa ggctgatagt 11100

ttagatgaaa agagagcatg atattttaag ccctgagact ggataatatc acctatagaa 11160

agactatata gagataagag aggtggggaa caagtaaaag ctgcgggaca ctcctaaatt 11220

tagagtcaaa tttagagcag aaaatactag caaaggggac tgaaaagcgg tggccaattg 11280

agcttcaaat gcaagtgaaa gtgtgttgtg tgtacattta tcatctcatg gcacaggaaa 11340

aacgtgattt aaggagaagg aagcgatcca atgggaagaa gagatccaat ggatcctcta 11400

tcacgaagat attgagataa gaaccaatat ggatttgcac ccactgcatt tgcagccttg 11460

aggtcataag catcctcagg aaaatgcacc aggtcagtgc taaggactgg agtcaataca 11520

gactggattc aggagaaaaa gtgaggcagg gaatcaaagg gagcaaacac agaaaactcc 11580

ttccaaaatc tggctgcaaa ttcttttcat agaaagaatt ctctcaggag aaatagggtt 11640

aacaccagaa aggaatatag tccaagatct ttttggttgt tttttgtttt gttttttggg 11700

gtggttttta ttttgttttt gtttagtttt gttgttgttt gtttgtttgt ttttgagacg 11760

gagtcactgt tgcccaggct ggagtgcagt ggtgcgatct cagctcactg caacctccgc 11820

cttccggtgt tcaagaggtc ttcctgcctc agcttcccaa gtagctggga ttgcaagcgc 11880

gtgccatcac gtccagctaa tttttgtatt tttagtagag atgaggtttc accatgttgg 11940

ccagtctggt ctcaaactcc tggcctcaag tgatccactt gcctcagctt ccctaaatgt 12000

tgggattaca ggtgtgagcc acggcacctg gccaaggatg gtttcttttt aaagggaggg 12060

gatatgattt atgtaatgtt tatgtaatag tgagaatgat ctagtagaaa taaaatcttg 12120

agggtttgtt tttggcagaa aatggaagga caaatgcagg gggaaagttt gtgggaagat 12180

gagagggcat cctgggccaa tgcagggagg gaaattattg gaaagaacaa gaagtcttta 12240

ttcacagtga cagaggcagg tagactgagg gtcaggggag gaccggctga aaatttcaca 12300

tctagttatt accattttct cattataatg tcagtcaagg gcatctgctg agagtgggaa 12360

aaacagagta aatgaggtac tttttggaga aagcaggact gaaataatta aggtgtggga 12420

tagcgagtgc gctggagaag tagagtaacc taatgaggaa ctgctgagtt ttcatttgaa 12480

atctgtggtc ataaattcaa ttgcaaccct cgataattgt gaaagtcact tccgctctct 12540

gagccgtact ttcctacctg ttacacaagg acagcacttg tcccaaagca agggtattgt 12600

aatcagagat gataaaatat atagtgactg gcacatatta aggactcacc aaatagcaga 12660

cctcataatc agtacaggta tcatctgtgg ttcgcagttc tcctccttac attacaacta 12720

gggtggaccc aaaactttgc aatttttgcc ttgttaattt gtcctcacct gtcattgcac 12780

aatcatcctt ccttgttgca tcaacatttt tttttttctg agacagagtc tcgctctttc 12840

acccaggctg gagtgctgtg gcacgatctt ggctcactgc aacctctgcc tcccagactc 12900

aagtgattct cctgcctcag cctcccaagt aactgggatt acaggcaccc accaccatgc 12960

ccagctaatt tttgtatttt tagtagagac agggtttcac catgttggcc aggctggttt 13020

caaattcctg gcctcaagtg atccacctgt cttggcctcc caaagtgcta ggattacagg 13080

cgtgagccac tgcgcccagc ctgtatcaac attttaaaat gaatttgaaa ttatggatag 13140

atcatgtctt ctaagtgtct tgtagaatgt ttggagtgtg gatagagaaa atctgagtct 13200

gttgaggaac tatggggtgg aaaagggtta acagcacaat tctgttgtaa tcaatctggc 13260

tgagctccaa ttctgtctct actctttcta cctggtaacg ttgaacaatt aacgcagccc 13320

atctaagtct taacttcctc atctgtacaa tggttatact aattatcagg ctgctgcaaa 13380

agtacttgtg gtttttgata ttattttcaa tggcaaaaac agcgttactt ttgcaccaac 13440

ctaatatcac taagattaat caatattcac tgtgtctcag gagtgctcag tgttcacaca 13500

tattaacaca gcccatccac agatcctgtg agaggcgttg atagggttaa aggaaaagct 13560

cttggtgtgt gggtaatgtt cataagtaga aacaactatt gctatgagca tcgttcttgt 13620

cctgaccttg aacttcataa tggattgagc cccacgacac agcacacagc ctggtgaggg 13680

ccaagagttc ccatttgtca tagatatact cagtctagcc taaaagattt ccacatctat 13740

tctcacttaa tcctcacctg aaagttagac ttcaccataa aattgtcctc gttctgcaga 13800

gcagaaaact gaggctcaaa gagatatcac tgactaaaaa ctcatagtga ggagacatca 13860

cagatgagat tagaagagag atgtagctga cttcagagcc cagtatatat atatatttta 13920

gacagagtct cgctctgtcc cccaggctgg agtgcagtgg cacggctcac tgcaacctcc 13980

gcctcccggg ttcaagcgat tctcctgcct cagcctcccg agtagctggg actgcaagtg 14040

catgccacca ggcccggcta attttttgta ttttcggtag agacggggct tcaccgtatt 14100

gcccagggtg gtctggaact cctgacctca ggcaatccac cccccttggc ctcccaaagt 14160

gttgggatta caggcgtgag ccaccgtatc tggctgagcc cagtattctt tgtaacagtg 14220

actctcaatt gggggtgact ttgcctccga ggggaccttt gacaatgttt atggaacctg 14280

ttgattgtca tttctggaga aggagttgct actggtgtct gtggatagag gccagggggg 14340

ctgctcaaca tcctactgtg tccagaacag tctgccacaa cgaagaatta cccagaccca 14400

gatgtcaaag gtgccaaggt tgagaaaccc tgagctacat taaataacgt cccatggaat 14460

acctcagtga aactcactgg taatagcagg aagaaaagga ggaaataagg aaaaggaaga 14520

aggctaaaaa tcaccagact gaggcatagc cctatcctag taatcttggg ggaatccctt 14580

tcaaaggcag gacttccgta tgcaaaggca gtggctctgg agttgttcat ctcggtttcc 14640

gtatcttgct gcctccactg actggctggg catgatgttg aaaaaaacta cagaagtccc 14700

cgggccatga attcccccag atgtgatggc tgtttcccaa tgttattatg agcattagat 14760

gtgaacaagt gctatggaat tgctatttct cttaaagatg ctgaatagcc cagtctgaga 14820

tgattctcaa attctcatgg ccatagcaaa tcatcctggc ttttacacat acagattcta 14880

gaatgagatt tatggatata attgtttttc atttttatta taagttcagg ggtatatgtg 14940

caggtttgtt atacaggtaa acttgtatcc tggtgttttc ttgtacagat tatttcatca 15000

ctgagatatt aagcctaata cccactactt atttttcctg atcctgtccc tcccctcacc 15060

ctccaccttc cggtaggccc cagtgttccc ctctttgtgt ccatgtgttc tcatcattta 15120

cctcccattt gtaaatgaga acatgtggta tttggttttc tgttcctgca ttagtttgct 15180

aaggataatg aatacaattt taacaggcag cctggatgtt tctgataaag tccacagacc 15240

acggcataag aaaatgtgct caggtgacgt gggactttgt gttgaactgg atacaaatat 15300

tacagatgtg atccggacca gaggcaggga ctacatgttt tgggtttttg ttcttaaaag 15360

aaaaaaacaa aaacaaaaaa acttgatctg agaagctctg aagggtcata ttcttacaat 15420

gttattgtcc acaatttagt cacgtgtcca ctaccaaagc aatcccttgc aaagtaaaag 15480

ggattctggt tagtgctaat tagactagtc aggatttact cccaacatgg agaaaagaca 15540

ccttctctgc agggtgagta actaaacaca attgtggttt ttcaagcaga gaagaatggg 15600

gaaccgttgc tgggtgggca accagcaata tctgtcaagc gtaagggttt agaagtcaga 15660

ccatgccaaa ctgaagtcct gaatctatta tctactacct aagtgacctt caacacattt 15720

cacaattgcc ctgagctttc atttattcta atgaaaacta gaagtaatga atattacata 15780

gctattacat aggccaaacc ggatcccata tgtagtaatt aacacaatgc atagtctcta 15840

ataagaactt cacaagtatt aatacaactt attattctta tagtctttat cccacagaca 15900

ccaaagctat acaaatcggc actgattatt tgcattccaa acagagaaga gctccacatc 15960

aagcagggac agggaaaagc agagggtaga aagcagagat ggaactctct ttgctgactt 16020

cctctagtgc agaatcctca ggatataaca tactactaca tgttaccagt gactcctcaa 16080

aattctcttc aacaataacc tcaagccaaa ccctcaaact ttatcttgag tactagccaa 16140

gcaataggtc aagggttctc acagtttgag ggacaggagt tgggagactg gaagaacctg 16200

gggctgaaag agattgcaga gcttgccagc atgtttgcat tgaatggtgg gagacacaga 16260

tgtgagcctt ctgattctga gtcatgtgcc ttcctcacaa tgctgaggct gcctatgctc 16320

aattttccat tgtccataca acataagttc agtatgagcc ttgtaatcct cttggtgaag 16380

tgatcagccc gataagcgaa atgcatattg ataatgatgg gaatatggag gactatccct 16440

tcccaaagga gcatccaaga gaatgccaat tctatagcac cgttgtcaac aaggcttggt 16500

agatagagtg ggtctcctgg taggagtggg agctttagtg gaagttgatg tgagctataa 16560

atacagatgc tgtaagatgg ttccacagct gagaaatggc cattgttgga atgtttcagg 16620

tgctgctggc aagatggaaa ccaacttctc cactcctctg aatgaatatg aagaagtgtc 16680

ctatgagtct gctggctaca ctgttctgcg gatcctccca ttggtggtgc ttggggtcac 16740

ctttgtcctc ggggtcctgg gcaatggctt tgtgatctgg gtggctggat tccggatgac 16800

acgcacagtc accaccatct gttacctgaa cctggccctg gctgactttt ctttcacggc 16860

cacattacca ttcctcattg tctccatggc catgggagaa aatggccttt ttggctggtt 16920

cctgtgtaag ttaattcaca tcgtggtgga catcaacctc tttggaagtg tcttcttgat 16980

tggtttcatt gcactggacc gctgcatttg tgtcctgcat ccagtctggg cccagaacca 17040

ccgcactgtg agtctggcca tgaaggtgat cgtcggacct tggattcttg ctctagtcct 17100

taccttgcca gttttcctct ttttgactac agtaactatt ccaaatgggg acacatactg 17160

tactttcaac tttgcatcct ggggtggcac ccctgaggag aggctgaagg tggccattac 17220

catgctgaca gccagaggga ttatccggtt tgtcattggc tttagcttgc cgatgtccat 17280

tgttgccatc tgctatgggc tcattgcagc caagatccac aaaaagggca tgattaaatc 17340

cagccgtccc ttacgggtcc tcactgctgt ggtggcttct ttcttcatct gttggtttcc 17400

ctttcaactg gttgcccttc tgggcaccgt ctggctcaaa gagatgttgt tctatggcaa 17460

gtacaaaatc attgacatcc tggttaaccc aacgagctcc ctggccttct tcaacagctg 17520

cctcaacccc atgctttacg tctttgtggg ccaagacttc cgagagagac tgatccactc 17580

cctgcccacc agtctggaga gggccctgtc tgaggactca gccccaacta atgacacggc 17640

tgccaattct gcttcacctc ctgcagagac tgagttacag gcaatgtgag gatggggtca 17700

gggatatttt gagttctgtt catcctaccc taatgccagt tccagcttca tctacccttg 17760

agtcatattg aggcattcaa ggatgcacag ctcaagtatt tattcaggaa aaatgctttt 17820

gtgtccctga tttggggcta agaaatagac agtcaggcta ctaaaatatt agtgttattt 17880

tttgtttttt gacttctgcc tataccctgg ggtaagtgga gttgggaaat acaagaagag 17940

aaagaccagt ggggatttgt aagacttaga tgagatagcg cataataagg ggaagacttt 18000

aaagtataaa gtaaaatgtt tgctgtaggt tttttatagc tattaaaaaa aatcagatta 18060

tggaagtttt cttctatttt tagtttgcta agagttttct gtttcttttt cttacatcat 18120

gagtggactt tgcattttat caaatgcatt ttctacatgt attaagatgg tcatattatt 18180

cttcttcttt tatgtaaatc attataaata atgttcatta agttctgaat gttaaactac 18240

tcttgaattc ctggaataaa ccacacttag tcctgatgta ctttaaatat ttatatctca 18300

caggagttgg ttagaatttc tgtgtttatg tttatatact gttatttcac tttttctact 18360

atccttgcta agttttcata gaaaataagg aacaaagaga aacttgtaat ggtctctgaa 18420

aaggaattga gaagtaattc ctctgattct gttttctggt gttatatctt tattaaatat 18480

tcagaaaaat tcaccagtga a 18501

<210> 5
<211> 1938
<212> DNA

<213>  Homo sapiens

<400>  5
gttgctccac aggaagccaa gaagcacaca ggaaaaggag cttagctgct ggtgctgctg      60

gcaagatgga aaccaacttc tccactcctc tgaatgaata tgaagaagtg tcctatgagt     120

ctgctggcta cactgttctg cggatcctcc cattggtggt gcttggggtc acctttgtcc     180

tcggggtcct gggcaatggg cttgtgatct gggtggctgg attccggatg acacgcacag     240

tcaccaccat ctgttacctg aacctggccc tggctgactt ttctttcacg gccacattac     300

cattcctcat tgtctccatg gccatgggag aaaaatggcc ttttggctgg ttcctgtgta     360

agttaattca catcgtggtg gacatcaacc tctttggaag tgtcttcttg attggtttca     420

ttgcactgga ccgctgcatt tgtgtcctgc atccagtctg ggcccagaac caccgcactg     480

tgagtctggc catgaaggtg atcgtcggac cttggattct tgctctagtc cttaccttgc     540

cagttttcct ctttttgact acagtaacta ttccaaatgg ggacacatac tgtactttca     600

actttgcatc ctggggtggc acccctgagg agaggctgaa ggtggccatt accatgctga     660

cagccagagg gattatccgg tttgtcattg gctttagctt gccgatgtcc attgttgcca     720

tctgctatgg gctcattgca gccaagatcc acaaaaaggg catgattaaa tccagccgtc     780

ccttacgggt cctcactgct gtggtggctt ctttcttcat ctgttggttt ccctttcaac     840

tggttgccct tctgggcacc gtctggctca aagagatgtt gttctatggc aagtacaaaa     900

tcattgacat cctggttaac ccaacgagct ccctggcctt cttcaacagc tgcctcaacc     960

ccatgcttta cgtctttgtg ggccaagact tccgagagag actgatccac tccctgccca    1020

ccagtctgga gagggccctg tctgaggact cagccccaac taatgacacg gctgccaatt    1080

ctgcttcacc tcctgcagag actgagttac aggcaatgtg aggatggggt cagggatatt    1140

ttgagttctg ttcatcctac cctaatgcca gttccagctt catctaccct tgagtcatat    1200

tgaggcattc aaggatgcac agctcaagta tttattcagg aaaaatgctt ttgtgtccct    1260

gatttggggc taagaaatag acagtcaggc tactaaaata ttagtgttat tttttgtttt    1320

ttgacttctg cctataccct ggggtaagtg gagttgggaa atacaagaag agaaagacca    1380

gtggggattt gtaagactta gatgagatag cgcataataa ggggaagact ttaaagtata    1440

aagtaaaatg tttgctgtag gttttttata gctattaaaa aaaatcagat tatggaagtt    1500

ttcttctatt tttagtttgc taagagtttt ctgtttcttt ttcttacatc atgagtggac    1560

tttgcatttt atcaaatgca ttttctacat gtattaagat ggtcatatta ttcttcttct    1620

tttatgtaaa tcattataaa taatgttcat taagttctga atgttaaact actcttgaat    1680

tcctggaata aaccacactt agtcctgatg tactttaaat atttatatct cacaggagtt    1740

ggttagaatt tctgtgttta tgtttatata ctgttatttc actttttcta ctatccttgc    1800

taagttttca tagaaaataa ggaacaaaga gaaacttgta atggtctctg aaaaggaatt 1860

gagaagtaat tcctctgatt ctgttttctg gtgttatatc tttattaaat attcagaaaa 1920

attcaccagt gaagccaa 1938

<210> 6
<211> 351
<212> PRT
<213> Homo sapiens

<400> 6

Met Glu Thr Asn Phe Ser Thr Pro Leu Asn Glu Tyr Glu Glu Val Ser
1               5                   10                  15

Tyr Glu Ser Ala Gly Tyr Thr Val Leu Arg Ile Leu Pro Leu Val Val
            20                  25                  30

Leu Gly Val Thr Phe Val Leu Gly Val Leu Gly Asn Gly Leu Val Ile
        35                  40                  45

Trp Val Ala Gly Phe Arg Met Thr Arg Thr Val Thr Thr Ile Cys Tyr
    50                  55                  60

Leu Asn Leu Ala Leu Ala Asp Phe Ser Phe Thr Ala Thr Leu Pro Phe
65                  70                  75                  80

Leu Ile Val Ser Met Ala Met Gly Glu Lys Trp Pro Phe Gly Trp Phe
                85                  90                  95

Leu Cys Lys Leu Ile His Ile Val Val Asp Ile Asn Leu Phe Gly Ser
            100                 105                 110

Val Phe Leu Ile Gly Phe Ile Ala Leu Asp Arg Cys Ile Cys Val Leu
        115                 120                 125

His Pro Val Trp Ala Gln Asn His Arg Thr Val Ser Leu Ala Met Lys
    130                 135                 140

Val Ile Val Gly Pro Trp Ile Leu Ala Leu Val Leu Thr Leu Pro Val
145                 150                 155                 160

Phe Leu Phe Leu Thr Thr Val Thr Ile Pro Asn Gly Asp Thr Tyr Cys
                165                 170                 175

Thr Phe Asn Phe Ala Ser Trp Gly Gly Thr Pro Glu Glu Arg Leu Lys
            180                 185                 190

Val Ala Ile Thr Met Leu Thr Ala Arg Gly Ile Ile Arg Phe Val Ile

195                 200                 205

Gly Phe Ser Leu Pro Met Ser Ile Val Ala Ile Cys Tyr Gly Leu Ile
    210                 215                 220

Ala Ala Lys Ile His Lys Lys Gly Met Ile Lys Ser Ser Arg Pro Leu
225                 230                 235                 240

Arg Val Leu Thr Ala Val Val Ala Ser Phe Phe Ile Cys Trp Phe Pro
                245                 250                 255

Phe Gln Leu Val Ala Leu Leu Gly Thr Val Trp Leu Lys Glu Met Leu
            260                 265                 270

Phe Tyr Gly Lys Tyr Lys Ile Ile Asp Ile Leu Val Asn Pro Thr Ser
        275                 280                 285

Ser Leu Ala Phe Phe Asn Ser Cys Leu Asn Pro Met Leu Tyr Val Phe
    290                 295                 300

Val Gly Gln Asp Phe Arg Glu Arg Leu Ile His Ser Leu Pro Thr Ser
305                 310                 315                 320

Leu Glu Arg Ala Leu Ser Glu Asp Ser Ala Pro Thr Asn Asp Thr Ala
                325                 330                 335

Ala Asn Ser Ala Ser Pro Pro Ala Glu Thr Glu Leu Gln Ala Met
            340                 345                 350

<210>  7
<211>  2019
<212>  DNA
<213>  Homo sapiens

<400>  7
cgatccaatg ggaagaagag atccaatgga tcctctatca cgaagatatt gagataagaa      60

ccaatatgga tttgcaccca ctgcatttgc agccttgagg tcataagcat cctcaggaaa     120

atgcaccagg tgctgctggc aagatggaaa ccaacttctc cactcctctg aatgaatatg     180

aagaagtgtc ctatgagtct gctggctaca ctgttctgcg gatcctccca ttggtggtgc     240

ttggggtcac ctttgtcctc ggggtcctgg gcaatgggct tgtgatctgg gtggctggat     300

tccggatgac acgcacagtc accaccatct gttacctgaa cctggccctg gctgactttt     360

ctttcacggc cacattacca ttcctcattg tctccatggc catgggagaa aaatggcctt     420

ttggctggtt cctgtgtaag ttaattcaca tcgtggtgga catcaacctc tttggaagtg     480

tcttcttgat tggtttcatt gcactggacc gctgcatttg tgtcctgcat ccagtctggg     540

cccagaacca ccgcactgtg agtctggcca tgaaggtgat cgtcggacct tggattcttg 600

ctctagtcct taccttgcca gttttcctct ttttgactac agtaactatt ccaaatgggg 660

acacatactg tactttcaac tttgcatcct ggggtggcac ccctgaggag aggctgaagg 720

tggccattac catgctgaca gccagaggga ttatccggtt tgtcattggc tttagcttgc 780

cgatgtccat tgttgccatc tgctatgggc tcattgcagc caagatccac aaaaagggca 840

tgattaaatc cagccgtccc ttacgggtcc tcactgctgt ggtggcttct ttcttcatct 900

gttggtttcc ctttcaactg gttgcccttc tgggcaccgt ctggctcaaa gagatgttgt 960

tctatggcaa gtacaaaatc attgacatcc tggttaaccc aacgagctcc ctggccttct 1020

tcaacagctg cctcaacccc atgctttacg tctttgtggg ccaagacttc cgagagagac 1080

tgatccactc cctgcccacc agtctggaga gggccctgtc tgaggactca gccccaacta 1140

atgacacggc tgccaattct gcttcacctc ctgcagagac tgagttacag gcaatgtgag 1200

gatggggtca gggatatttt gagttctgtt catcctaccc taatgccagt tccagcttca 1260

tctacccttg agtcatattg aggcattcaa ggatgcacag ctcaagtatt tattcaggaa 1320

aaatgctttt gtgtccctga tttggggcta agaaatagac agtcaggcta ctaaaatatt 1380

agtgttattt tttgtttttt gacttctgcc tataccctgg ggtaagtgga gttgggaaat 1440

acaagaagag aaagaccagt ggggatttgt aagacttaga tgagatagcg cataataagg 1500

ggaagacttt aaagtataaa gtaaaatgtt tgctgtaggt tttttatagc tattaaaaaa 1560

aatcagatta tggaagtttt cttctatttt tagtttgcta agagttttct gtttcttttt 1620

cttacatcat gagtggactt tgcattttat caaatgcatt ttctacatgt attaagatgg 1680

tcatattatt cttcttcttt tatgtaaatc attataaata atgttcatta agttctgaat 1740

gttaaactac tcttgaattc ctggaataaa ccacacttag tcctgatgta ctttaaatat 1800

ttatatctca caggagttgg ttagaatttc tgtgtttatg tttatatact gttatttcac 1860

tttttctact atccttgcta agttttcata gaaaataagg aacaaagaga aacttgtaat 1920

ggtctctgaa aaggaattga gaagtaattc ctctgattct gttttctggt gttatatctt 1980

tattaaatat tcagaaaaat tcaccagtga aaaaaaaaa 2019

<210> 8
<211> 351
<212> PRT
<213> Homo sapiens

<400> 8

Met Glu Thr Asn Phe Ser Thr Pro Leu Asn Glu Tyr Glu Glu Val Ser
1               5                   10                  15

Tyr Glu Ser Ala Gly Tyr Thr Val Leu Arg Ile Leu Pro Leu Val Val

20                  25                  30

Leu Gly Val Thr Phe Val Leu Gly Val Leu Gly Asn Gly Leu Val Ile
        35                  40                  45

Trp Val Ala Gly Phe Arg Met Thr Arg Thr Val Thr Thr Ile Cys Tyr
    50                  55                  60

Leu Asn Leu Ala Leu Ala Asp Phe Ser Phe Thr Ala Thr Leu Pro Phe
65                  70                  75                  80

Leu Ile Val Ser Met Ala Met Gly Glu Lys Trp Pro Phe Gly Trp Phe
                85                  90                  95

Leu Cys Lys Leu Ile His Ile Val Val Asp Ile Asn Leu Phe Gly Ser
            100                 105                 110

Val Phe Leu Ile Gly Phe Ile Ala Leu Asp Arg Cys Ile Cys Val Leu
        115                 120                 125

His Pro Val Trp Ala Gln Asn His Arg Thr Val Ser Leu Ala Met Lys
    130                 135                 140

Val Ile Val Gly Pro Trp Ile Leu Ala Leu Val Leu Thr Leu Pro Val
145                 150                 155                 160

Phe Leu Phe Leu Thr Thr Val Thr Ile Pro Asn Gly Asp Thr Tyr Cys
                165                 170                 175

Thr Phe Asn Phe Ala Ser Trp Gly Gly Thr Pro Glu Glu Arg Leu Lys
            180                 185                 190

Val Ala Ile Thr Met Leu Thr Ala Arg Gly Ile Ile Arg Phe Val Ile
        195                 200                 205

Gly Phe Ser Leu Pro Met Ser Ile Val Ala Ile Cys Tyr Gly Leu Ile
    210                 215                 220

Ala Ala Lys Ile His Lys Lys Gly Met Ile Lys Ser Ser Arg Pro Leu
225                 230                 235                 240

Arg Val Leu Thr Ala Val Val Ala Ser Phe Phe Ile Cys Trp Phe Pro
                245                 250                 255

Phe Gln Leu Val Ala Leu Leu Gly Thr Val Trp Leu Lys Glu Met Leu
            260                 265                 270

Phe Tyr Gly Lys Tyr Lys Ile Ile Asp Ile Leu Val Asn Pro Thr Ser
        275                 280                 285

Ser Leu Ala Phe Phe Asn Ser Cys Leu Asn Pro Met Leu Tyr Val Phe
    290                 295                 300

Val Gly Gln Asp Phe Arg Glu Arg Leu Ile His Ser Leu Pro Thr Ser
305                 310                 315                 320

Leu Glu Arg Ala Leu Ser Glu Asp Ser Ala Pro Thr Asn Asp Thr Ala
                325                 330                 335

Ala Asn Ser Ala Ser Pro Pro Ala Glu Thr Glu Leu Gln Ala Met
            340                 345                 350

<210>  9
<211>  18
<212>  DNA
<213>  Artificial Sequence

<220>
<223>  F3 Primer

<400>  9
gcggatgaag actggtgt                                                    18

<210>  10
<211>  18
<212>  DNA
<213>  Artificial Sequence

<220>
<223>  R3 Primer

<400>  10
gttgctcgta tttagggc                                                    18

## Description

The present invention is related to FPR2 receptor agonist aptamers and uses thereof in the prophylaxis and/or treatment of a disease, condition or pathology characterised by a decrease or inhibition of the expression of the FPR2 receptor, and/or a decrease in or locking of the activation of the FPR2 receptor and/or an absence/low amount of the natural ligand of the FPR2 receptor.

### BACKGROUND OF THE INVENTION

G protein-coupled receptors (GPCRs) constitute the largest family of membrane-bound receptors in the human genome. Although they recognise a wide variety of ligands, such as peptides, small organic molecules, calcium ions and even photons, they have a related structure that includes 7 transmembrane domains. GPCRs modulate a wide variety of physiological processes and numerous mutations have been described in the genes encoding these disease-associated receptors. Therefore, they constitute the main therapeutic targets of the drugs that currently exist (approximately 35%).

The rhodopsin-like GPCRs (GPCRA) is the most studied group from the structural and functional point of view, and it is divided into 19 subfamilies. They represent approximately 80% of all GPCRs. Within this family are FPRs (Formyl Peptide Receptors). The N-formylated peptides produced by Gram-negative bacteria were the first identified ligands for the FPRs FPR1 and FPR2 (FPRL1). At present, multiple agonists and antagonists for these receptors are known, thus having a great variety of functions in different systems. Specifically, FPR2 is expressed in a great diversity of cells, including astrocytes, epithelial cells, hepatocytes, endothelial cells, neuroblastoma and leukocytes, so it is involved in different physiological and pathological processes, such as the inflammatory response, healing, angiogenesis and cancer. Known FPR2 agonists include the antimicrobial peptide LL37, the therapeutic use of which is protected by patent US20100210539A1 for possible applications in cancer, autoimmune diseases, fibrotic diseases, inflammatory diseases, neurodegenerative diseases, infectious diseases, lung, heart, vascular and metabolic diseases. Patent EP1358888A1 also describes the use of LL37/hCAP18 as an inducer of angiogenesis and patent US7452864B2 describes the use of LL37 and peptides derived therefrom for use in healing. Several studies have been carried out to test the benefits of the LL37 peptide in the treatment of chronic wounds of various aetiologies. However, peptides in general, and the LL37 in particular, have serious drawbacks in relation to their low activity and availability at the wound site, thus requiring repetitive applications and high doses of the peptides that can lead to toxicity and undesirable side effects.

Therefore, in view of the aforementioned drawbacks, there is a need in the state of the art to provide new molecules capable of interacting with, and specifically binding to, rhodopsin-like G protein-coupled receptors and activate the cascade of reactions associated thereto, useful in the treatment of different pathologies associated with defects in these receptors, specifically in the treatment of wound healing.

### DESCRIPTION OF THE INVENTION

The present inventors have developed nucleic acid aptamers that act as agonist molecules of rhodopsin-like GPCR receptors (GPCRA), particularly, of the FPR2 receptor *(Formyl peptide receptor 2).*

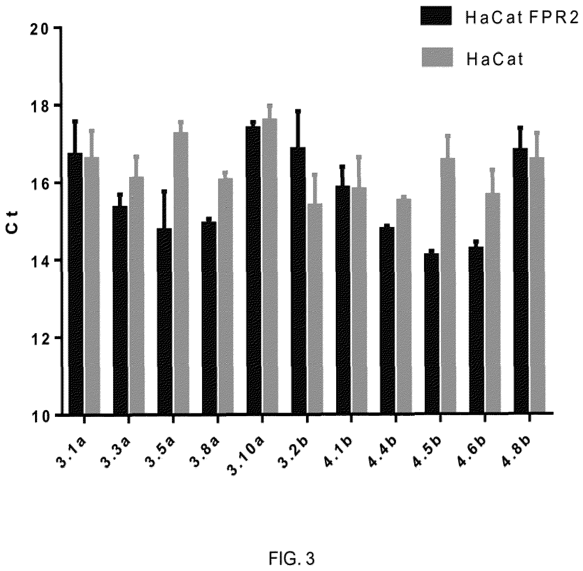

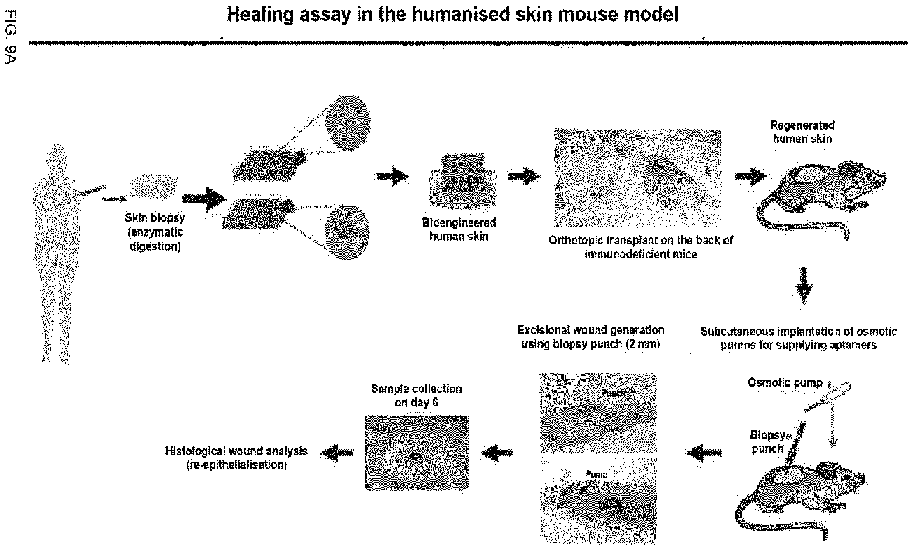

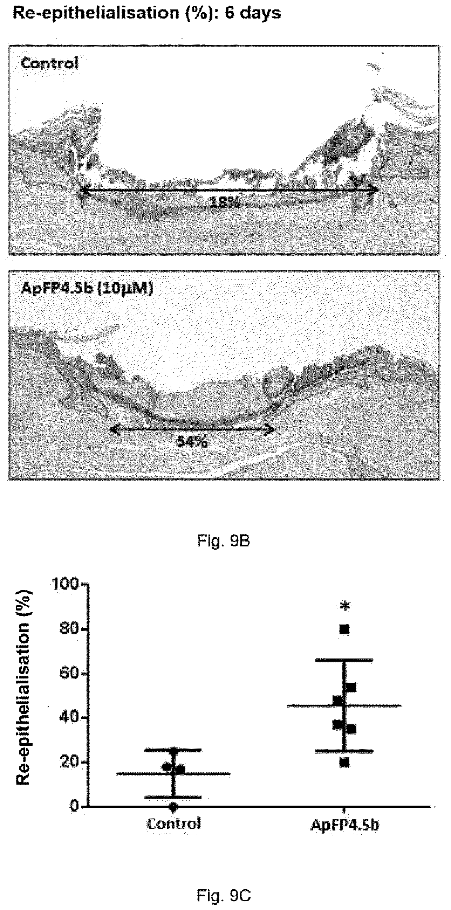

The aptamers of the present invention were obtained by means of the SELEX method (Systematic Evolution of Ligands by Exponential Enrichment), after which the affinity, cellular location and activity of each one of them was analysed (see Example of the present description, Figures 3 and 5 to 8). Thus, aptamers were found to be able to inhibit cAMP production as mediated by forskolin, and to activate the migration of the human keratinocyte line HaCaT by stably expressing the receptor FPR2. In addition, an *in vivo* experiment was also carried out with one of the aptamers of the invention (specifically the aptamer ApFP4.5b) wherein the pro-healing action was observed, inducing an improvement in the re-epithelialisation process (see Figure 9).

These aptamers, due to the three-dimensional structure thereof, are able to recognise receptors with high affinity and specificity, specifically bind to them, and activate the cascade of reactions associated with them. Therefore, the aptamers provided by the present invention constitute tools with great diagnostic and therapeutic value. In addition, being single-stranded nucleic acid molecules, they have a series of properties and features that differentiate them from other agonists such as small molecules and antibodies. Among them, it is worth noting that they are not immunogenic, have low cytotoxicity and are easily eliminated, which makes the aptamers of the present invention a highly relevant therapeutic tool for all those diseases characterised in that the FPR2 receptor is totally/partially inhibited or inactivated, or in those diseases in which there is no availability of endogenous receptor ligands or there is availability of endogenous ligand, but an extra supply of exogenous ligand can be beneficial. Additional advantages are the reduced production costs and the fact that when chemically synthesised they do not have any differences between batches.

Thus, based on these aptamers, the inventors have developed a series of inventive aspects which will be described in detail below.

### Aptamer of the invention

In one aspect, the present invention is related to a nucleic acid aptamer that is able to specifically bind to the FPR2 receptor and activate it, hereinafter "aptamer of the invention", comprising a nucleotide sequence with a sequence identity of at least 70% with a nucleotide sequence selected from the group consisting of the sequences SEQ ID NO: 1, SEQ ID NO: 2 and SEQ ID NO: 3.

The term "aptamer", in the context of the present invention, is related to single stranded nucleic acid chains that adopt a specific tertiary structure that enables them to bind to molecular targets with high specificity and affinity, comparable to that of monoclonal antibodies, through interactions other than classical Watson-Crick base pairing, such as electrostatic and ionic interactions.

The term "nucleic acid", in the context of the present invention, refers to any type of nucleic acid, such as DNA and RNA, and variants thereof, such as peptide nucleic acid (PNA), locked nucleic acid (LNA), as well as combinations thereof, modifications thereof, that include modified nucleotides, etc. The terms "nucleic acid" and "oligonucleotide" and "polynucleotide" are interchangeably used in the context of the present invention. Nucleic acids can be purified from natural sources, be produced using recombinant expression systems and optionally, purified, chemically synthesised, etc. Where appropriate, for example, in the case of chemically synthesised molecules, nucleic acids can comprise nucleoside analogues such as analogues having chemically modified bases or sugars, skeletal modifications, etc. A nucleic acid sequence is represented in the 5'-3' direction unless otherwise indicated.

In the present invention, the term "FPR2" refers to the FPR2 cell receptor, acronym for *Formyl peptide receptor 2,* located on the cell surface of a wide variety of cell types, such as neutrophils, monocytes, macrophages, immature dendritic cells, B cells, T cells, epithelial cells, astrocytes, hepatocytes and endothelial cells, as well as in cells of different animal species. Other names for this receptor include, but are not limited to, ALXR, FMLP-R-II, FMLPX, FPR2A, FPRH1, FPRH2, FPRL1, HM63 and LXA4R.

In humans, the FPR2 receptor comprises 351 amino acids and is encoded by the *FPR2* gene (NCBI Reference Sequence: NC_000019.10, ACCESSION NC_000019 Region: 51752026..51770526, (SEQ ID NO: 4)) within an open reading frame without introns. The gene forms a *cluster* with *FPR1* and *FPR3* genes on chromosome 19q.13.3. The transcription of the FPR2 gene gives rise to two transcripts:
- NCBI, NM_001005738.2 (SEQ ID NO: 5), the translation of which gives rise to the FPR2 receptor comprising the sequence SEQ ID NO: 6 (NCBI, NP_001005738.1), and
- NCBI, NM_001462.3 (SEQ ID NO: 7), the translation of which gives rise to the FPR2 receptor comprising the sequence SEQ ID NO: 8 (NCBI, NP_001453.1).

In a particular embodiment, the FPR2 receptor is a mouse, rat, rabbit, pig, cat, dog, horse or non-human primate receptor. In another particular embodiment of the aptamer of the invention, the FPR2 receptor is the human FPR2 receptor.

As explained earlier, the aptamer of the invention is able to specifically bind to the FPR2 receptor, in other words, it is able to recognise it in a specific manner and bind thereto to activate it just as the natural ligand of said receptor would.

"Specific binding" or "specific binding to the FPR2 receptor", in the present invention is understood as the non-covalent physical association between two molecules, the aptamer of the invention and the FPR2 receptor. The binding between the aptamer of the invention and the FPR2 receptor is considered specific if the binding strength between the two is at least 10 times, at least 15 times, at least 20 times, at least 25 times, at least 50 times, at least 75 times or at least 100 times greater than the binding strength between the aptamer of the invention and an irrelevant molecule. The binding between the aptamer of the invention and the FPR2 receptor is also considered specific if the equilibrium dissociation constant Kd is 10⁻³ M or less, 10⁻⁴ M or less, 10⁻⁵ M or less, 10⁻⁶ M or less, 10⁻⁷ M or less, 10⁻⁸ M or less, 10⁻⁹ M or less, 10⁻¹⁰ M or less, 10⁻¹¹ M or less, or 10⁻¹² M or less under the conditions used, for example, under physiological conditions, cell culture conditions or conditions that enable cell survival.

The ability of the aptamer of the invention to specifically bind to the FPR2 receptor can be determined by means of a variety of assays that are available in the state of the art. Preferably, the specific binding ability to the FPR2 receptor of the aptamer of the invention is determined by means of *in vitro* binding assays, such as the Enzyme-Linked OligoNucleotide Assay, ELONA), the Enzyme-Linked Aptamer Sorbent Assay, ELASA), precipitation and quantitative PCR (qPCR), or by means of fluorescence techniques such as aptahistochemistry, aptacytochemistry, fluorescence microscopy or flow cytometry. Likewise, both the specific binding ability to the FPR2 receptor and the affinity of the aptamer for the FPR2 receptor can be determined by means of techniques widely known to those skilled in the art, such as the gel mobility shift assay, surface plasmon resonance, SPR), kinetic capillary electrophoresis and fluorescence binding assay. Briefly, the fluorescence binding assay consists of the incubation of magnetic beads coated with the FPR2 receptor with different concentrations (for example, from 0 to 100 nM) of the marked aptamer of the invention (for example, with carboxyfluorescein, FAM), and the subsequent elution and detection of the bound aptamers, dissociation constants (Kd) are calculated by means of nonlinear fit analysis.

As explained earlier at the beginning of this description, the aptamer of the invention is an agonist of the FPR2 receptor, in other words, the aptamer of the invention acts as an activator of said receptor. In the present invention, "activation of the FPR2 receptor" is understood as the presence of, or the increase of, the signal mediated by the FPR2 receptor with respect to the inactivated state thereof. The activity of a receptor is considered to be increased by an activator or agonist when the activity thereof or the signal mediated by the receptor is greater than the activity or signal mediated by the natural ligand of the receptor. The increase in activity can be at least 1%, at least 5 %, at least 10 %, at least 20 %, at least 30 %, at least 40 %, at least 50 %, at least 60 %, at least 70 %, at least 80 %, at least 90% or at least 100% greater than the activity of the receptor when it binds to the natural ligand thereof. The natural ligands of the FPR2 receptor are widely known in the state of the art. Natural ligands for FPR2 include N-formylated peptides and other non-formylated, non-endogenous bacterial peptides/proteins, such as the gp41 and gp120 proteins of the human immunodeficiency virus type 1 (HIV-1), the Helicobacter pylori peptide Hp2-20, the serum amyloid A (SAA) acute phase protein, the peptide sequence 106-126 of the PrP protein, the 42 amino acid variant of the amyloid peptide Aβ (Aβ42), Humanin, Annexin A1 and peptides derived from this protein (Ac2-26 and Ac9-25), the antimicrobial peptide LL37, uPAR-derived peptides (uPAR D2D388-274 and uPAR84-95), FAM3D, CCL23 and an N-terminal fragment derived from it (SHAAGtide), the vasoactive intestinal neuropeptide VIP, Lipoxin A4 (LXA4), Resolvin D1 and oxLDL. The ability of an aptamer of the invention to activate the FPR2 receptor can be determined by means of a wide variety of assays available in the state of the art. Preferably, the ability of the aptamer of the invention to activate FPR2 is determined by *in vitro* assays with cells expressing recombinant FPR2 and a reporter gene the expression of which is associated with the activation of said recombinant receptors. The person skilled in the art will recognise that there are multiple variants of this method, depending on the cell and the recombinant gene used. An example of this assay is contained in the Examples of the present invention ("Materials and Methods" section). Other techniques available include determining the levels of inflammatory cytokines, such as, for example, IL-1β, IL-2, IL-4, IL-5, IL-6, IL-10, IL-12, IL-18, IL-20, IL-31, IL-32, TNF, GM-CSF, CCL2, CCL3, CCL4, CCL5, CCL7, CCL20, CXCL1, CXCL4, CXCL8 and CXCL10 among others, released by different cell types that express the FPR2 receptor.

The aptamer of the invention that is able to specifically bind to the FPR2 receptor and activate said receptor FPR2 comprises a nucleotide sequence having a sequence identity of at least 70% with a nucleotide sequence selected from the group consisting of the sequences SEQ ID NO: 1, SEQ ID NO: 2 and SEQ ID NO: 3.

In the present invention, "identity" or "sequence identity" is understood as the degree of similarity between two nucleotide or amino acid sequences obtained by aligning the two sequences. Depending on the number of common residues between the aligned sequences, a degree of identity will be obtained expressed as a percentage. The degree of identity between two nucleotide/amino acid sequences can be determined by means of standard methods, for example, by means of standard sequence alignment algorithms known in the state of the art, such as BLAST [Altschul S.F. et al. Basic local alignment search tool. J Mol Biol. 1990 5 Oct; 215(3):403-10]. BLAST programs, for example, BLASTN, BLASTX, and TBLASTX, BLASTP and TBLASTN, are in the public domain on *The National Center for Biotechonology Information* (NCBI) website.

The person skilled in the art understands that mutations in the nucleotide sequence of genes that give rise to conservative substitutions of amino acids in positions that are not critical for the functionality of the protein, are evolutionarily neutral mutations that do not affect the overall structure or functionality thereof, giving rise to variants functionally equivalent to the original nucleotide sequence. Said variants fall within the scope of the present invention. Thus, all those nucleotide sequences that have at least 70% identity with the nucleotide sequences SEQ ID NO: 1, SEQ ID NO: 2 or SEQ ID NO: 3 and that also have the same function as said sequences, in other words, that are able to specifically bind to the FPR2 receptor and activate said FPR2 receptor, are functionally equivalent variants that are also envisaged within the scope of protection of the present invention.

In the present invention, "functionally equivalent variant" is understood to relate to aptamers with sequences substantially similar to the sequences SEQ ID NO: 1, SEQ ID NO: 2 or SEQ ID NO: 3 that maintain their ability to specifically bind and activate the FPR2 receptor. A functionally equivalent variant of the aptamer of the invention can be a nucleic acid sequence derived from the sequence SEQ ID NO: 1, SEQ ID NO: 2 or SEQ ID NO: 3 comprising the addition, substitution or modification of one or more nucleotides. By way of illustration, functionally equivalent variants of the aptamer of the invention include sequences comprising the addition of 1 nucleotide, 2 nucleotides, 3 nucleotides, 4 nucleotides, 5 nucleotides, 10 nucleotides, 15 nucleotides, 20 nucleotides, 25 nucleotides, 30 nucleotides, 35 nucleotides, 40 nucleotides, 45 nucleotides, 50 nucleotides, 60 nucleotides, 70 nucleotides, 80 nucleotides, 90 nucleotides, 100 nucleotides, 150 nucleotides, 200 nucleotides, at least 500 nucleotides, at least 1000 nucleotides or more at the 5' end of the sequence SEQ ID NO: 1, SEQ ID NO: 2 or SEQ ID NO: 3, and/or comprising the addition of 1 nucleotide, 2 nucleotides, 3 nucleotides, 4 nucleotides, 5 nucleotides, 10 nucleotides, 15 nucleotides, 20 nucleotides, 25 nucleotides, 30 nucleotides, 35 nucleotides, 40 nucleotides, 45 nucleotides, 50 nucleotides, 60 nucleotides, 70 nucleotides, 80 nucleotides, 90 nucleotides, 100 nucleotides, 150 nucleotides, 200 nucleotides, at least 500 nucleotides, at least 1000 nucleotides or more at the 3' end of the sequence SEQ ID NO: 1, SEQ ID NO: 2 or SEQ ID NO: 3, and that maintain an ability to specifically bind and activate the FPR2 receptor of at least 50%, at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, or at least 100% of its ability to specifically bind and activate the FPR2 receptor.

The present invention also includes aptamers comprising nucleotide sequences with a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with the sequences SEQ ID NO: 1, SEQ ID NO: 2 or SEQ ID NO: 3, and that maintain an ability to specifically bind and activate the FPR2 receptor of at least 50%, at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, or at least 100% of its ability to specifically bind and activate the FPR2 receptor.

Thus, in a particular embodiment of the invention, the aptamer comprises a nucleotide sequence having a sequence identity of at least 70, 75, 80, 85, 90, 91, 92, 93, 94, 95, 96, 97, 98 or 99% with the sequence SEQ ID NO: 1, SEQ ID NO: 2 or SEQ ID NO: 3. In a more particular embodiment, the aptamer comprises a nucleotide sequence that has a 100% sequence identity to the sequence SEQ ID NO: 1, SEQ ID NO: 2 or SEQ ID NO: 3. In another even more particular embodiment, the aptamer of the invention consists of the sequence SEQ ID NO: 1, SEQ ID NO: 2 or SEQ ID NO: 3.

The aptamers of the present invention are nucleic acid aptamers, which can be DNA or RNA. In a particular embodiment, the aptamer of the invention is a DNA aptamer. Nevertheless, it is also envisaged in the present invention that the aptamer may be made up of nucleic acid variants and analogues and combinations thereof. Examples of nucleic acid variants and analogues and combinations thereof, which include, but are not limited to, modified nucleic acid skeletons, replacement bonds, modified nucleotides, and ribose or deoxyribose analogues, modified nucleotides, etc. that are able to specifically bind and activate the FPR2 receptor by at least 50%, at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, or at least 100% of the ability to specifically bind and activate the FPR2 receptor of the aptamer comprising the sequence SEQ ID NO: 1, SEQ ID NO: 2 or SEQ ID NO: 3. Non-limiting examples of nucleic acid variants and analogues include, but are not limited to, APN, ANB and ATN.

The term "nucleic acid variant" or "nucleic acid analogue", in the context of the present invention, refers to nucleic acid variants and analogues including, but are not limited to, modified nucleic acid skeletons, replacement bonds, modified nucleotides, and ribose or deoxyribose analogues. For example, nucleic acid variants according to the present invention may comprise structures with synthetic skeletons analogous to the typical phosphodiester skeleton. These include, but are not limited to, phosphorothioate, phosphorodithioate, methylphosphonate, phosphoramidate, alkyl phosphotriester, sulphamate, 3'-thioacetal, methylene (methylimine), 3'-N-carbamate, morpholino carbamate and peptide nucleic acids (PNA), methylphosphonate bonds or alternating methylphosphonate and phosphodiester and benzylphosphonate bonds.

Variants of a nucleic acid can also contain one or more "replacement" bonds, as is generally understood in the art. Some of these replacement bonds are apolar and contribute to providing the aptamer with an ability to diffuse through the membranes. These "replacement" bonds are defined herein as conventional alternative bonds such as phosphorothioate or phosphoramidate, and are synthesised as described in commonly available literature. Alternative binding groups include, in a non-limiting manner, embodiments wherein a moiety of formula P(O)S, ("lioalo"), P(S)S ("dilioalo"), P(O)NR' P(O)R', P(O)OR', CO, or CONR' wherein R' is H (or a salt) or a 1-12 C atoms alkyl group and R6 is a 1-9 C atoms alkyl group, that bind to adjacent nucleotides through -S-o of -0-. The present invention also envisages the use of replacement bonds that include non-phosphorous-based internucleotide bonds such as 3'-thioformacetal, (-S-CH2-O-), formacetal (-O-CH2-O-) and 3' amine internucleotide bonds (-NH-CH2-CH:d). One or more replacement bonds can be used in the aptamers of the invention in order to further facilitate binding to the FPR2 receptor, or to increase the stability of aptamers against nucleases, as well as to confer permeation capacity. Not all bonds within the same aptamer have to be identical and therefore, the present invention envisages aptamers with all identical bonds as well as aptamers with a variation in the composition of their bonds.

Likewise, nucleic acid variants according to the present invention may also contain analogous forms of ribose or deoxyribose which are well known in the art, including but not limited to 2' substituted sugars such as 2'-O-methyl ribose, 2'-fluororibose or 2'-azido-ribose, carbocyclic analogues of sugars, α-anomeric sugars, epimeric sugars such as arabinose, xyloses or lyxoses, pyranose sugars, furanose sugars and sedoheptuloses. Nucleic acids may also contain threose nucleic acid (TNA), also referred to as alpha-threofuranosyl oligonucleotides). Particularly, replacement at the 2' position of the furanose residue is particularly important with respect to improving nuclease stability.

The term "nucleotide", in the context of the present invention, refers to the monomers that make up nucleic acids. Nucleotides are made up of a pentose, a nitrogenous base and a phosphate group, and are bound by phosphodiester bonds. The nucleotides that are part of DNA and RNA differ in the pentose, this being deoxyribose and ribose respectively. Nitrogenous bases, in turn, are divided into purine nitrogenous bases, which are adenine (A) and guanine (G), and into pyrimidine nitrogenous bases, which are thymine (T), cytosine (C) and uracil (U). Thymine only appears in DNA, whereas uracil only appears in RNA. The present invention envisages the use of modified nucleotides in the aptamer of the invention. The term "modified nucleotide" in the context of the present invention, refers to known natural nucleotide analogues, with similar or improved binding properties. Analogous forms of purines and pyrimidines are well known in the art, and include, but are not limited to, aziridinylcytosine, 4-acetylcytosine, 5-fluorouracil, 5-bromouracil, 5-carboxymethylaminomethyl-2-thiouracil, 5-carboxymethylaminomethyluracil, inosine, N₆-isopentenyladenine, 1-methyladenine, 1-methylpseudouracil, 1-methylguanine, 1-methylinosine, 2,2-dimethylguanine, 2-methyladenine, 2-methylguanine, 3-methylcytosine, 5-methylcytosine, N₆₋methyladenine, 7-methylguanine, 5-methylaminomethyluracil, 5-methoxyaminomethyl-2-thiouracil, beta-D-manosylqueosine, 5-methoxyuracil, 2-methylthio-N-6-isopentenyladenine, uracyl-5-oxyacetic acid methyl ester, pseudouracil, queosine, 2-thiocytosine, 5-methyl-2-thiouracil, 2-thiouracil, 4-thiouracil, 5-methyluracil, uracil-5-oxyacetic acid, and 2,6-diaminopurine. In addition to the above modified nucleotides, nucleotide residues lacking a purine or a pyrimidine can also be included in the present invention.

In addition to the previous variants, nucleic acid variants comprised by the invention also include APN, ANB and 5'-5' or 3'-3' chains. The term "peptide nucleic acid" or "PNA", in the context of the present invention, refers to an oligonucleotide the skeleton of which is composed of repeating units of N- (2-aminoethyl) glycine bound by peptide bonds, wherein the different nitrogenous bases are bound to the main chain by a methylene bond (-CHT) and a carbonyl group (-(C=O)). The term "locked nucleic acid" or "LNA", in the context of the present invention, refers to a modified RNA nucleotide the ribose residue of which is modified with an additional bond that connects oxygen at 2' to carbon at 4', locking ribose in the 3'-endo conformation. The term "5'-5' chain" or "3'-3' chain", in the context of the present invention, refers to oligonucleotides in which the nucleotide at the 3' or 5' ends, respectively, is reversed.

The aptamer of the invention comprises a variable size of nucleotides. In a particular embodiment, it is between 76 and 200 nucleotides in size, preferably between 76 and 150 nucleotides, more preferably, between 76 and 100 nucleotides, even more preferably, the aptamer of the invention comprises 76 nucleotides.

The production of the aptamer of the invention can be carried out following conventional methods in the art. Non-limiting examples of aptamer production techniques include enzymatic techniques, such as transcription, recombinant expression systems and standard solid phase (or solution phase) chemical synthesis, that are commercially available. Where appropriate, for example, in the event that the aptamer of the invention comprises nucleic acid variants as described above, nucleotide analogues such as analogues having chemically modified bases or sugars, skeletal modifications, etc., the aptamer of the invention will be produced by means of chemical synthesis. Alternatively, recombinant expression will be the preferred technique for the production of aptamers when they are 200 nucleotides or longer in length. Aptamers produced by any of the above techniques can optionally be purified by means of methods well known in the art.

### Complex of the invention

As the person skilled in the art will appreciate, the features of small size, stability and easy production of the aptamer of the invention, cause it to present itself bound to a second molecule. This is particularly advantageous when the second molecule is a functional group. The result of the binding of the aptamer of the invention and a functional group is a complex that exhibits the combination of functions of both, in other words, a complex that is able to specifically bind and activate the FPR2 receptor and with the activity associated with the functional group.

Therefore, in another aspect, the present invention relates to a complex, hereinafter referred to as the "complex of the invention", comprising the aptamer of the invention and a functional group.

The term "aptamer" has been described in detail in the previous inventive aspect (aptamer of the invention) and its definitions and particular features also apply to the complex of the invention.

The term "functional group", in the context of the present invention, refers to molecules capable of exerting at least one function. Said function includes, but are not limited to, the ability to specifically bind to the FPR2 receptor, or to other receptors for rhodopsin-like GPCRs, such as the *Formyl Peptide Receptors* or FPRs, and activate them, the ability to be detectable, both directly and indirectly, the ability to induce cell death, etc. As the person skilled in the art will understand, a functional group may have one or more functions associated with it. Non-limiting examples of functional groups include detectable reagents and drugs. These functional groups can act as imaging agents, drugs, etc. Therefore, in a particular embodiment, the functional group is a detectable reagent, a drug or a nanoparticle.

The term "detectable reagent", in the context of the present invention, is related to reagents that are able to be directly or indirectly detectable. Examples of detectable reagents indicated for the present invention include, but are not limited to, radionuclides, fluorophores, proteins and haptens.

In a preferred embodiment, the detectable reagent is a radionuclide. To this end, appropriate radionuclides have utility in diagnostic imaging techniques, such as radioimmunodiagnostic techniques and positron emission tomography (PET). Non-limiting examples of radionuclides include gamma emitting isotopes, such as 9^{9m}Tc, ¹²³I and ¹¹¹In, which can be used in radioscintigraphy using gamma cameras or single photon emission computerised tomography, as well as positron emitters, such as ¹⁸F, ⁶⁴Cu, ⁶⁸ Ga, ⁸⁶Y, ¹²⁴I, ²¹³Bi and ²¹¹At, that can be used in PET, or beta emitters, such as ¹³¹I, ⁹⁰Y, ^{99m}Tc, ¹⁷⁷Lu and ⁶⁷Cu. The person skilled in the art will appreciate that radionuclides can also be used for therapeutic purposes.

In another preferred embodiment, the detectable reagent is a fluorophore. The term "fluorophore", in the context of the present invention, refers to a fluorescent chemical compound that can emit light after being excited by light of a different wavelength. Suitable fluorophores in the present invention include, but are not limited to, Cy3, Cy2, Cy5, the Alexa Fluor^{®} family of fluorescent markers (Molecular Probes, Inc.), carboxyfluorescein (FAM) and fluorescein isothiocyanate (FITC).

In another preferred embodiment, the detectable reagent is a protein. The term "protein", in the context of the present invention, refers to macromolecules consisting of one or more amino acid chains. Proteins are responsible for carrying out a diverse group of cellular functions based on their ability to bind other molecules in specific ways. Proteins can bind to other proteins as well as to small substrate molecules. Non-limiting examples of proteins suitable for the purposes of the present invention include, but are not limited to, enzymes, fluorescent proteins, luminescent proteins and antigens.

In an even more preferred embodiment, the protein is an enzyme. The term "enzyme", in the context of the present invention, refers to a protein that functions as a highly selective catalyst, accelerating both the rate and the specificity of the metabolic reaction for which it is specific. Non-limiting examples of enzymes suitable for the invention include, but are not limited to, horseradish peroxidase, HRP) and alkaline phosphatase. As the person skilled in the art will appreciate, the enzymes suitable for use in the present invention are indirectly detectable due to the ability thereof to catalyse the modification of a substrate into a compound detectable by colourimetry, chemiluminescence or fluorimetry. Examples of suitable substrates include, but are not limited to, p-Nitrophenyl phosphate (P-NPP), 2,2'-azinobis [3-ethylbenzothiazoline-6-sulphonic acid] (ABTS), o-phenylenediamine (OPD), and 3,3', 5,5'-tetramethylbenzidine (TMB).

Bioluminescent proteins or photoproteins are a particular case of oxidative enzymes that are capable of carrying out a chemical reaction of their specific prosthetic groups, resulting in an emission of light without the need for prior excitation. Non-limiting examples of bioluminescent proteins include firefly luciferase, luciferase *Renilla* and the aequorin.

In another even more preferred embodiment, the protein is a fluorescent protein. The term "fluorescent protein", in the context of the present invention, is related to a protein with the ability to emit light when excited at a suitable wavelength for excitation. Non-limiting examples of fluorescent proteins that can be used in the complex of the invention include, but are not limited to, GFP, GFPuv, BFP, CFP, YFP, EBFP2, mCerulean, mCerulean3, mVenus, mTurquoise, T-Sapphire, citrine, amFP486, zFP506, zFP538, drFP, DsRed, mCherry, dTomato, mTFP1, TagRFP-T, mKO2, mRuby, mKate, mAmetrine, REACh, R-phycoerythrin (R-PE) and Allophycocyanin (APC).

In another even more preferred embodiment, protein is a luminescent protein. The term "luminescent protein", in the context of the present invention, refers to a protein capable of emitting light when excited at a suitable wavelength for excitation.

In another even more preferred embodiment, the protein is an antigen. The term "antigen", in the context of the present invention, refers to a molecule that induces an immune response in the body. Therefore, an antigen can be used to generate an antibody that specifically recognises and binds thereto. Non-limiting examples of antigens include, among others, tumour antigens, such as carcinoembryonic antigen (CEA), HER2, prostate specific antigen (PSA) and plasminogen tissue activator and the recombinant variants thereof such as Activase^{®}, as well as bacterial antigens, allergens, etc. As the person skilled in the art will appreciate, antigens suitable for use in the present invention are indirectly detectable due to their ability to be specifically recognised by an antibody.

In another preferred embodiment, the detectable reagent is a hapten. The term "hapten", in the context of the present invention, refers to a group of chemical compounds of small molecular size (generally, less than 10.000 Da) that are antigenic but unable to induce a specific immune reaction by themselves. The chemical coupling of a hapten to a large immunogenic protein, called carrier, generates an immunogenic hapten-carrier conjugate that is able to induce a specific immune reaction. Non-limiting examples of vitamins include biotin (vitamin B7), digoxigenin, dinitrophenol (DNP) and nitro-iodophenol (NIP). In a more preferred embodiment, the vitamin is biotin. The term "biotin", in the context of the present invention, refers to a heat-stable vitamin, soluble in water and alcohol, also called vitamin H and vitamin B7, characterised in that it specifically binds to avidin with the highest affinity reported to date of Kd = 10¹⁵ M. As the person skilled in the art will appreciate, biotin is indirectly detectable due to the ability thereof to be specifically recognised by avidin or variants thereof such as streptavidin and neutravidin.

In another particular embodiment, the functional group is a drug. The term "drug", in the context of the present invention, refers to a chemical used in the treatment, cure or prevention of a disease, condition, or pathology characterised by a decrease or inhibition of the expression of the FPR2 receptor, and/or a decrease in the activation of the FPR2 receptor. It is also related to that chemical substance used in the treatment, cure or prevention of a disease, condition, or pathology characterised by the unavailability of endogenous FPR2 receptor ligands or, even with endogenous ligand availability, an extra supply of exogenous ligand could be beneficial. Thus, these receptors have been shown to be involved in cancer development and progression. In addition FPR2 is involved in the control of inflammatory processes, exerting pro- or anti-inflammatory functions depending on the cellular context and the availability of ligands. Chronic inflammation is in many cases responsible for the pathogenesis of many human diseases, wherein FPR2 receptor agonists could show protective and restorative effects by means of the stimulation of the anti-inflammatory pathways. In addition, FPR2 is expressed in epithelial and endothelial cells, playing a fundamental role in the processes of wound repair and restoration of the integrity of the barrier function, by activating epithelial cell proliferation and migration processes and promoting neo-angiogenesis. Therefore, the diseases or conditions that can be treated with FPR2 aptamers are all those included in the following categories: cancer, inflammatory diseases, autoimmune diseases, metabolic diseases, neurodegenerative diseases, fibrotic diseases, infectious diseases, cardiovascular diseases and epithelial tissue pathologies.

The present invention envisages that the drug is a nucleic acid. Thus, in a preferred embodiment the drug is a nucleic acid. Nucleic acids suitable as drugs in the context of the complex of the invention include antisense RNA, antisense DNA and small interfering RNA.

The present invention envisages that the drug is a peptide. Thus, in a preferred embodiment the drug is a peptide. The term "peptide", in the context of the present invention, is related to a short amino acid chain bound by peptide bonds. The peptide will comprise at least 2 amino acids, at least 3 amino acids, at least 4 amino acids, at least 5 amino acids, at least 10 amino acids, at least 15 amino acids, at least 20 amino acids, at least 30 amino acids, at least 40 amino acids, at least 50 amino acids, at least 60 amino acids, or at least 70 amino acids. Suitable for the purposes of this invention are, among others, peptides that are able to bind to a target and to induce or inhibit cell signalling. The term "target binding peptide", in the context of the present invention, refers to a peptide comprising a target binding region. The term "signalling peptide", in the context of the present invention, refers to a peptide with the ability to cause cell signalling, such as cell receptor agonist peptides. Suitable amino acid sequences for the binding of target molecules include molecular recognition consensus sequences that is well known in the art.

In another particular embodiment, the functional group is a nanoparticle. As used in the present description, the term "nanoparticle" refers to colloidal systems of the spherical, cylindrical, polyhedral, etc. type, or similar shapes, that are less than or equal to 1 µm in size, which can be found individually or forming organised structures (dimer, trimers, etc.), dispersed in a fluid (aqueous solution). In a particular embodiment, nanoparticles suitable for putting the invention into practice have a size smaller than 1 µm, generally between 1 and 99 nanometres (nm), typically between 5 and 500 nm, preferably between about 10 and 150 nm. In a particular embodiment, nanoparticles have a mean diameter between 2 and 50 nm, preferably between 5 and 20 nm. The mean particle diameter is the maximum mean particle dimension, understanding that nanoparticles do not necessarily have to be spherical. Suitable nanoparticles for use in the present invention include polymeric nanoparticles, lipid nanoparticles and metallic nanoparticles.

The polymeric nanoparticles are formed by a polymeric matrix that is anchored to the aptamer of the invention. Examples of biocompatible polymers that can be used in the context of the present invention include, but are not limited to, polyethylenes, polycarbonates and polyanhydrides.

Alternatively, the nanoparticles that act as functional groups can be lipid nanoparticles such as a liposome or a micelle. The formation of micelles and liposomes from, for example, lipids that form vesicles, is widely known in the state of the art.

Likewise, the nanoparticle can be a metallic nanoparticle. The term "metallic nanoparticle" is related to nanoparticles comprising a metal and comprises the property known as surface plasmon phenomenon. The surface plasmon of a metal can be measured by means of any spectroscopic technique of the state of the art, such as surface plasmon resonance spectroscopy.

In a particular embodiment, the nanoparticle is a silica mesoporous magnetic nanoparticle.

Nanoparticles can be functionalised by adding a coating to the surface thereof. For biological applications, the coating surface should be polar to provide high aqueous solubility and prevent aggregation of the nanoparticles.

The aptamer of the invention can bind to the nanoparticle by means of covalent bonds, preferably on the surface of the nanoparticle.

The binding between an aptamer of the invention and a functional group to generate the complex of the invention can be carried out by means of conjugation techniques widely known to the persons skilled in the art. The result is a covalent bond between the aptamer of the invention and the functional group. The conjugation may involve the binding of primary amines from the 3' or 5' ends of the aptamer of the invention to the functional group during the chemical synthesis of the aptamer. Alternatively, the conjugation can be carried out by means of conventional cross-linking reactions, which have the advantage of the much higher chemical reactivity of primary alkyl amine tags versus the aryl amines of the nucleotides themselves. Conjugation methods are well known in the art and are based on the use of cross-linking reagents. Cross-linking reagents contain at least two reagent groups, that target groups such as primary amines, sulphydryls, aldehydes, carboxyls, hydroxyls, azides and so on, in the molecule to be conjugated. Cross-linking agents differ in chemical specificity, the length of the spacer arm, the composition of the spacer arm, cleavage spacer arm, and structure. For example, the conjugation of complexes according to the invention can be carried out directly or through a linking moiety, through one or more non-functional groups on the aptamer and/or the functional group, such as amine groups, carboxyl, phenyl, thiol or hydroxyl. More selective bonds can be achieved by using a heterobifunctional binder. Conventional binders can be used, such as diisiocyanates, diisothiocyanates, bis (hydroxysuccinimide) esters, carbodiimides, maleimide-hydroxysuccinimide esters, glutaraldehyde and the like, ° hydrazines and hydrazides, such as butyric acid 4-(4-N-maleimidophenyl) hydrazide (MPBH).

Another approach consists of the marking of aptamers during PCR synthesis by using marked primers, for example, with a fluorophore. To that end, there are various commercial houses available to the person skilled in the art.

In addition, in the particular embodiment wherein the functional group is a radionuclide, the binding between an aptamer according to the invention and the radionuclide can be carried out by means of chemical coordination, wherein the aptamer atoms involved in the binding donate electrons to the radionuclide. Coordination reactions are widely known in the art and will depend on the radionuclide and reagent group involved in the aptamer.

### In vitro uses of the aptamer of the invention

The aptamer of the invention is able to recognise the FPR2 receptor, specifically bind thereto, and activate the signalling associated with said receptors, just like its natural ligands do. This ability of the aptamer of the invention can then be used to detect the presence of the receptor by detecting the aptamer-receptor binding.

Therefore, in another aspect, the present invention relates to the *in vitro* use of the aptamer of the invention to detect the FPR2 receptor.

Thus, the ability of an aptamer according to the invention to specifically bind to the FPR2 receptor can be exploited for the indirect detection of said receptor. For this purpose, the person skilled in the art will recognise that a subsequent detection of said aptamer is necessary. Aptamer detection techniques are well known in the art and include, for example, the use of antibodies or specific probes against the aptamer. As such, once the aptamer of the invention is bound to the FPR2 receptor, an antibody or probe specific for the aptamer would be applied, which in turn can be marked with a detectable reagent, or that can be detected indirectly by means of an antibody or secondary probe. The technique used to detect the FPR2 receptor will then depend on the type of detectable reagent, being able to be techniques that are based, for example, on fluorimetry, colourimetry or radioactivity.

The term "probe" or "hybridisation probe", in the context of the present invention, refers to a DNA or RNA fragment of variable length, usually between 10 and 1,000 bases in length, which is used to detect the presence of single-stranded nucleic acids (DNA or RNA) that are complementary to the sequence in the probe. The probe hybridises to the target single-stranded nucleic acid, the base sequence of which enables base pairing due to the complementarity between the probe and the target nucleic acid. To detect probe hybridisation to the target sequence thereof, the probe is marked with a detectable reagent, such as a radionuclide, a fluorophore or digoxygenin, among others.

The detection of the binding of the FPR2 receptor with the aptamer of the invention can be carried out by means of *in vitro* binding assays, such as the Enzyme-Linked OligoNucleotide Assay, ELONA), the Enzyme-Linked Aptamer Sorbent Assay, ELASA), precipitation and quantitative PCR (qPCR), gel mobility shift assay, Western Blotting, surface plasmon resonance, SPR), kinetic capillary electrophoresis, the fluorescence binding assay, aptahistochemistry, aptacytochemistry, fluorescence microscopy or flow cytometry.

In another particular embodiment of the *in vitro* uses of the invention, the detection of the FPR2 receptor is performed by means of a method selected from the group consisting of ELONA, aptacytochemistry, aptahistochemistry and flow cytometry.

The term "ELONA" or "Enzyme-Linked OligoNucleotide Assay", in the context of the present invention, is related to a technique analogous to the Enzyme-Linked ImmunoSorbent Assay, ELISA), wherein the antibody that is used to detect the molecule of interest, in this case the FPR2 receptor, is exchanged for a detection aptamer specific for said molecule. The ELISA assay is based on the use of marked antigens or antibodies, for example with enzymes, so that the complexes formed between the target antigen and the marked antibody are enzymatically active complexes. Since one of the components, in this case the antigen, is immobilised on a carrier, the antigen:antibody complexes are immobilised to the carrier and, therefore, can be detected by adding a specific substrate for the enzyme. In the case of ELONA, the detection aptamer can be covalently bound to an enzyme, or it may itself be detected by an aptamer-specific secondary antibody that is enzyme-conjugated. Said enzyme catalyses the transformation of a specific substrate to produce a visible signal. This technique can be modified to exchange the enzyme for another detectable reagent, as can be a fluorophore. In this document the terms ELONA and ELASA, or Enzyme-Linked Aptamer Sorbent Assay are indistinctly used. In a preferred embodiment, the detection of the FPR2 receptor is carried out by means of ELONA.

Likewise, the terms "aptacytochemistry" and "aptahistochemistry", in the context of the present invention, refers to techniques that are analogous to immunocytochemistry and immunohistochemistry for the detection of the FPR2 receptor on cells and histological sections, respectively, wherein the antibody that is used to detect the molecule of interest, in this case the FPR2 receptor, is exchanged for an aptamer specific for said molecule. The detection aptamer can be covalently bound to an enzyme, or it may itself be detected by an aptamer-specific secondary antibody that is enzyme-conjugated. Said enzyme catalyses the transformation of a specific substrate to produce a visible signal. This technique can be modified to exchange the enzyme for another detectable reagent, as can be a fluorophore. In a preferred embodiment, the detection of the FPR2 receptor is carried out by means of aptacytochemistry. In another preferred embodiment, the detection of the FPR2 receptor is carried out by means of aptahistochemistry.

Alternatively, the person skilled in the art will recognise that these techniques (ELONA, aptacytochemistry, aptahistochemistry) can be adapted to exchange the detection antibody for an aptamer-specific probe.

The term "flow cytometry", in the context of the present invention, is related to a cell analysis technique that involves measuring the fluorescence and light scattering features that cells possess as they are passed through a beam of light. In addition to the scattering of light, if, prior to their analysis, the cells are placed in the presence of aptamers marked with fluorescent molecules, it is possible to evaluate which cells possess the antigens complementary to the aptamers used. Fluorescence detection is performed with flow cytofluorometers (known as "cytometers" or "FACS" ("Fluorescence-Activated Cell Sorter"). This technique, similarly to the previous ones, was initially developed for use with fluorescently marked antibodies but can be easily adapted for use with the aptamer of the invention.

As the person skilled in the art will appreciate, a complex comprising a nucleic acid aptamer that is able to specifically bind and activate the receptor, wherein said aptamer comprises a nucleotide sequence with a sequence identity of at least 70% with the sequence SEQ ID NO: 1, SEQ ID NO: 2 or SEQ ID NO: 3, and a detectable reagent is particularly advantageous for detecting the FPR2 receptor, since said detectable reagent makes it possible to detect the aptamer comprised in the complex when it is bound to the FPR2 receptor. The technique used to detect the FPR2 receptor will depend on the type of detectable reagent, being able to be techniques that are based, for example, on fluorimetry, colourimetry or radioactivity.

Thus, in another aspect, the present invention is related to a complex comprising (i) a nucleic acid aptamer that is able to specifically bind and activate the FPR2 receptor, comprising a nucleotide sequence with a sequence identity of at least 70% with the sequence SEQ ID NO: 1, SEQ ID NO: 2 or SEQ ID NO: 3, and (ii) a functional group to detect the FPR2 receptor. In a particular embodiment, the functional group is a detectable reagent. In another particular embodiment of the *in vitro* uses of the invention, the detection of the FPR2 receptor is performed by means of a method selected from the group consisting of ELONA, aptacytochemistry, aptahistochemistry and flow cytometry. The terms "aptamer", "FPR2", "complex", "functional group", "detectable reagent", "ELONA", "aptacytochemistry", "aptahistochemistry" and "flow cytometry" have been described in detail above and their definitions and particular features are included for reference herein.

Since ELISA techniques, immunocytochemistry, immunohistochemistry and flow cytometry are well known in the art, the person skilled in the art will be able to make the necessary adaptations to exchange the antibody for the aptamer or complex according to the invention without the need for undue experimentation.

As described earlier, the aptamer of the invention and the complex of the invention have the ability to specifically bind to and activate the FPR2 receptor. Therefore, in another aspect, the present invention relates to the *in vitro* use of the aptamer of the invention or of the complex of the invention to activate the FPR2 receptor. Both the aptamer and the complex of the invention are FPR2 receptor activators.

The detection of the binding of the FPR2 receptor with the aptamer of the invention can also be indirectly carried out by detecting the molecules produced as a consequence of said receptor-aptamer binding. Thus, as a result of said binding, an increase in the production of chemokines and chemokine receptors can be observed in monocytes and macrophages (for example, but not limited to, Gro-α, MDC, MCP-1, MIP-1α and MIP-1β), increased production of VEGF in monocytes and macrophages, noticeable increased intracellular Ca⁺ mobilisation to pertussis toxin, NF-κB nuclear translocation, phosphorylation of ERKs, induction of Bcl-xL and inhibition of caspase 3 activity in neutrophils, production of LTB4 in neutrophils, induction of phosphorylation and translocation of p47(phox) and activation of NADPH oxidase in human fibroblasts, etc. In human keratinocytes, as a result of the binding of the FPR2 receptor with the aptamer of the invention it can be observed, for example, but not limited to, an increased production of cytokines and chemokines, such as IL-6, IFN-β1, CXCL1, CXCL8/IL8, TNF-α, GM-CSF, IL-10, CXCL10/IP-10, MCP-1, CCL20/MIP3a, etc, induction of the transcription factors Snail and Slug, activation of MMP-2 and MMP-9, activation of MAPK and PI3k/Akt signalling pathways and increased phosphorylation of FAK and paxilin.

### In vitro methods of the invention

As previously explained, both the aptamer of the invention and the complex of the invention have the ability to specifically recognise and bind to the FPR2 receptor, resulting in the activation of said receptors.

Therefore, analogous to the *in vitro* uses described in previous paragraphs, the present invention is also related to an *in vitro* method for detecting the FPR2 receptor in an isolated biological sample from a subject, henceforth "first *in vitro* method of the invention", which comprises
(a) contacting said sample with the aptamer of the invention or the complex of the invention,
(b) separating the aptamer or complex that is not bound to the FPR2 receptor, and
(c) detecting the presence of the aptamer or complex that is bound to the FPR2 receptor present in the sample.

In the present invention, "sample" or "biological sample" is understood as the isolated cell culture or biological material from a subject. The biological sample can contain any biological material suitable to detect the desired biomarker and can comprise cells and/or non-cellular material from the subject. The sample can be isolated from any suitable tissue or biological fluid such as, for example, blood, plasma, serum, urine, cerebrospinal fluid (CSF), heart, brain. The samples used for the detection of the FPR2 receptor are preferably biological fluids.

The terms "biological fluid" and "biofluid" are interchangeably used herein and relate to aqueous fluids of biological origin. Biofluid can be obtained from any location (such as blood, plasma, serum, urine, bile, cerebrospinal fluid, vitreous or aqueous humour, or any body secretion), an exudate (such as fluid obtained from an abscess or any other infection or inflammation site), or fluid obtained from a joint (such as a normal joint or a joint affected by a disease such as rheumatoid arthritis). The biofluids used for the detection of the FPR2 receptor are preferably samples of blood, plasma, serum or cerebrospinal fluid.

The term "subject" or "individual" refers to a member of a mammalian species, and includes, but is not limited to pets, and primates including humans; the subject is preferably a human, man or woman, of any age or race.

The terms "aptamer" and "FPR2 receptor" have been described in detail earlier in the present description, their definitions and particularities being applicable to the present inventive aspect.

In a first step, the first method of the invention comprises contacting the isolated biological sample from the subject with the aptamer of the invention or the complex of the invention.

The aptamer or the complex of the invention is applied to the sample in a suitable buffer to enable the binding of the aptamer or the complex to the FPR2 receptor molecules that may be present in the sample. Non-limiting examples of suitable buffers to enable the binding of the aptamer or complex of the invention to FPR2 receptor molecules include, but are not limited to, PBS, TBS, phosphate buffer and citrate buffer. Preferably, these buffers contain 1mM MgCl₂. The amount of aptamer or complex of the invention required to detect the FPR2 receptor molecules present in the sample will depend on both the size of the sample and the amount of FPR2 receptor present therein, and it can be readily determined by means of optimisation methods that are common in the art. As a guide, the concentration of aptamer or complex of the invention is at least 1 fM, at least 10 fM, at least 100 fM, at least 1 pM, at least 10 pM, at least 100 pM, at least 1 nM, at least 10 nM, at least 100 nM, at least 1 µM, at least 10 µM, at least 100 µM or more. Preferably, the concentration of aptamer is between 100 fM and 1 µM, more preferably between 1 pM and 100 nM, even more preferably between 100 pM and 1 nM.

The aptamer or complex of the invention is incubated with the sample at a suitable temperature and for a time sufficient to enable the binding of the aptamer or complex to the FPR2 receptor molecules that may be present in the sample. Preferably, the temperature is between 20 °C and 37 °C. As a guide, the aptamer will be incubated with the sample for at least 5 minutes, at least 10 minutes, at least 15 minutes, at least 20 minutes, at least 30 minutes, at least 60 minutes, at least 120 minutes or more.

Once the aptamer or complex of the invention has bound to the FPR2 receptor molecules that may be present in the sample, in a second step of the first method of the invention, the sample is washed to remove aptamer or complex molecules that have not bound to the FPR2 receptor.

After washing, in a third step, the presence of the aptamer or the complex of the invention bound to the FPR2 receptor present in the sample is detected.

Since the aptamer of the invention is not itself a detectable molecule, the detection step is an indirect detection step through a second detectable molecule that specifically binds to the aptamer. Detection of the aptamer bound to the FPR2 receptor can be carried out with practically any known antibody or reagent that binds with high affinity to the aptamer of the invention. Nevertheless, the use of a specific antibody against the aptamer is preferable, for example, polyclonal serum, hybridoma supernatants, monoclonal or humanised antibodies and fragments thereof. Said aptamer-specific antibody is conveniently marked with a detectable reagent. The term "detectable reagent" has been described in detail above and its definition and features are included for reference herein. Said reagent can be detected by means of fluorimetry or colorimetry using equipment suitable for the type of reagents and the type of sample, which are known to the person skilled in the art. By way of example, the sample with the aptamer bound to the FPR2 receptor molecules present is incubated with an antibody specific to the aptamer that is enzyme-conjugated, under similar conditions to the conditions of incubation with the aptamer, and the FPR2-aptamer-antibody complexes are detected with the addition of a substrate that is converted by the enzyme to a detectable product, for example, by means of fluorimetry in a fluorescence microscope or by colorimetry in a spectrophotometer. Alternatively, detection can be carried out analogously by using an aptamer-specific probe suitably marked with a detectable reagent. The person skilled in the art will recognise that the first *in vitro* method of the invention can be carried out as part of detection techniques such as ELONA, ELASA, precipitation and qPCR, gel mobility shift assay, Western Blotting, surface plasmon resonance, kinetic capillary electrophoresis, fluorescence binding assay, aptahistochemistry, aptacytochemistry, fluorescence microscopy or flow cytometry.

In the event that the third step of the first method of the invention comprises the detection of the complex of the invention bound to the FPR2 receptor present in the sample, the detection of the complex according to the invention can be carried out with practically any known antibody or reagent that binds with high affinity to the aptamer of the invention or to the functional group. The detection of the aptamer of the invention has been described in detail in previous paragraphs. Likewise, in relation to the functional group, the detection can also be carried out with practically any known antibody or reagent that binds with high affinity to said functional group. For this reason, it is particularly convenient that the functional group is a detectable reagent.

In a particular embodiment, the functional group is a detectable reagent selected from the group consisting of radionuclides, fluorophores, proteins and haptens. The terms "radionuclide", "fluorophore", "detectable protein" and "hapten" have been described in detail above and their definitions and features are included for reference herein.

As the person skilled in the art will notice, the detectable reagents envisaged by the present invention can be divided into the reagents that are directly detectable by themselves, such as radionuclides or fluorophores, and reagents that are indirectly detectable, such as proteins or haptens. In a particular embodiment, the detectable reagent is a radionuclide and detection is carried out by detecting the radiation emitted by the radionuclide. This radiation will depend on the type of radionuclide, being able to be an emission of α particles, β particles or γ-type emission. To this end, detection techniques suitable for different radionuclides are widely known. By way of example, the emission emitted by ¹²³I can be detected by a gamma camera.

In another particular embodiment, the detectable reagent is a fluorophore and detection is carried out by detecting the fluorescence emitted by the fluorophore. The use of a fluorophore requires its prior excitation with a wavelength within the excitation spectrum thereof, which causes an emission at a different wavelength. The excitation and emission wavelengths of the fluorophores envisaged in the present invention are part of the state of the art. The emitted fluorescence can be detected, for example, by means of fluorometric techniques using a fluorescence spectrophotometer or a fluorescence microscope.

In another particular embodiment, the detectable reagent is a protein. This can be detected depending on the type of protein used. For example:
- an enzyme requires the addition of the specific substrate thereof that will be detectable by means of colorimetry, chemiluminescence or fluorimetry;
- a fluorescent protein, just like a fluorophore, requires excitation at a suitable wavelength to be detectable by means of fluorimetry
- an antigen or a hapten requires an antibody or other molecule that specifically recognises it. In order to be detected, said antibody or molecule specific for the antigen/hapten needs to be marked, for example, with an enzyme, and detection will depend on the type of marking.

The person skilled in the art will recognise that the third step of the first *in vitro* method of the invention can be carried out as part of detection techniques such as ELONA, ELASA, precipitation and qPCR, gel mobility shift assay, Western Blotting, surface plasmon resonance, kinetic capillary electrophoresis, fluorescence binding assay, aptahistochemistry, aptacytochemistry, fluorescence microscopy or flow cytometry. All these techniques have been explained in previous paragraphs of this description, and said explanations are applicable to the present inventive aspect. In a particular embodiment, detection of FPR2 is carried out by means of fluorescence.

In another aspect, the invention relates to an *in vitro* method to activate the FPR2 receptor in an isolated biological sample from a subject, henceforth "second *in vitro* method of the invention", comprising contacting said sample comprising the FPR2 receptor with the aptamer of the invention or the complex of the invention, under suitable conditions to activate the FPR2 receptor.

The terms "aptamer", "FPR2 receptor", "FPR2 activation", "sample" and "subject" have been described in detail above and their definitions and particular features are included for reference herein.

In a particular embodiment, the FPR2 receptor in the sample is comprised in living cells.

The term "suitable conditions to activate the FPR2 receptor", in the context of the present invention, refers to the incubation conditions that enable the binding of the aptamer or the complex of the invention to the FPR2 receptor and the subsequent activation thereof. These conditions include the composition of the buffer in which the aptamer or complex of the invention is applied on the sample, the amount of aptamer or complex, the incubation time and the incubation temperature. Non-limiting examples of suitable buffers to enable the binding of the aptamer of the invention to the FPR2 receptor and the subsequent activation thereof include, but are not limited to, PBS, TBS, phosphate buffer and citrate buffer. Preferably, these buffers contain 1 mM MgCl₂. The amount of aptamer or complex of the invention required to detect the FPR2 receptor molecules present in the sample will depend on both the size of the sample and the amount of FPR2 receptor molecules present therein, and it can be readily determined by means of optimisation methods that are common in the art. The concentrations of aptamer or complex of the invention have been previously described in the present description for other inventive aspects. The aptamer is incubated with the sample at a suitable temperature and for a sufficient time to enable the binding of the aptamer or the complex of the invention to the FPR2 receptor molecules that may be present in the sample. Preferably, the temperature is between 20 °C and 37 °C, more preferably 37 °C. As a guide, the aptamer will be incubated with the sample for at least 5 minutes, at least 10 minutes, at least 15 minutes, at least 20 minutes, at least 30 minutes, at least 60 minutes, at least 120 minutes, or more.

In a particular embodiment of the second method of the invention, the subject is a human. In another particular embodiment of the second method of the invention, the biological sample is blood, plasma, serum or cerebrospinal fluid. These terms have been defined in previous paragraphs of this description.

### Composition of the invention

As the person skilled in the art understands, both the aptamer of the invention and the complex of the invention can form part of a composition, more specifically, of a pharmaceutical composition if it is to be administered to an individual for therapeutic purposes.

Thus, in another aspect, the present invention relates to a pharmaceutical composition, hereinafter "pharmaceutical composition of the invention", comprising the aptamer of the invention and/or the complex of the invention, together with a support, a pharmaceutically acceptable excipient or vehicle.

As explained at the beginning of the present description, the aptamer of the invention is a nucleic acid aptamer that is able to specifically bind to the FPR2 receptor and activate it, comprising a nucleotide sequence with a sequence identity of at least 70% with the nucleotide sequence SEQ ID NO: 1, SEQ ID NO: 2 or SEQ ID NO: 3. If a functional group is bound to the aptamer of the invention, then we have the complex of the invention. The terms "aptamer", "FPR2 receptor", "activation", "sequence identity", "complex" and "functional group" have been previously defined, as well as the particular embodiments thereof, which are applicable to the present inventive aspect.

The composition of the invention may comprise more than one aptamer of the invention, wherein the aptamers comprise different nucleotide sequences between them. Thus, in a particular embodiment, the composition of the invention comprises aptamers comprising the nucleotide sequence SEQ ID NO: 1, and aptamers comprising the nucleotide sequence SEQ ID NO: 2. In another particular embodiment, the composition of the invention comprises aptamers comprising the nucleotide sequence SEQ ID NO: 1 and aptamers comprising the nucleotide sequence SEQ ID NO: 3. In another particular embodiment, the composition of the invention comprises aptamers comprising the nucleotide sequence SEQ ID NO: 2 and aptamers comprising the sequence SEQ ID NO: 3. In another particular embodiment, the composition of the invention comprises aptamers comprising the nucleotide sequence SEQ ID NO: 1, aptamers comprising the nucleotide sequence SEQ ID NO: 2 and aptamers comprising the nucleotide sequence SEQ ID NO: 3.Within the composition of the invention functionally equivalent variants of aptamers comprising the sequences SEQ ID NO: 1, SEQ ID NO: 2 or SEQ ID NO: 3 are also envisaged, wherein said variants have a sequence identity of at least 70%, with the sequence SEQ ID NO: 1, SEQ ID NO: 2 or SEQ ID NO: 3. The terms "sequence identity" and "functionally equivalent variant" have been defined for the aptamer of the invention. As the person skilled in the art understands, these particular embodiments also apply to the complex of the invention.

As used in the present description, the term "pharmaceutically acceptable support" or "pharmaceutically acceptable excipient" or "pharmaceutically acceptable solvent" is intended to include any and all solvents, dispersion media, coatings, antibacterial and antifungal agents, isotonic and absorption retarding agents, and the like, compatible with pharmaceutical administration. The use of such carriers and vehicles in pharmaceutically active substances is well known in the art. Except insofar as any conventional carrier is incompatible with the active compound, the use thereof in the compositions of the invention is envisaged. Acceptable vehicles, excipients, or acceptable stabilisers are not toxic to the subject at the doses and concentrations used, and include buffers such as phosphate, citrate, and other organic acids; antioxidants including ascorbic acid and methionine; preservatives (such as octadecyldimethylbenzyl ammonium chloride, hexamethonium chloride, benzalkonium chloride, benzethonium chloride; phenol, butyl or benzyl alcohol; alkyl parabens such as methyl or propyl paraben; catechol; resorcinol; cyclohexanol; 3-pentanol, and m-cresol); low molecular weight polypeptides (less than about 10 amino acids); proteins, such as serum albumin, gelatin, or immunoglobulins; hydrophilic polymers such as polyvinylpyrrolidone; amino acids such as glycine, glutamine, asparagine, histidine, arginine or lysine; monosaccharides, disaccharides, and other carbohydrates including glucose, mannose, or dextrins; chelating agents such as EDTA; sugars such as sucrose, mannitol, trehalosa or sorbitol; salt-forming counter ions such as sodium; metal complexes (for example, Zn-protein complexes); and/or non-ionic surfactants such as TWEEN^{™}, PLURONICS^{™} or polyethylene glycol (PEG).

Supplementary active compounds can also be incorporated into the pharmaceutical composition provided by the present invention. Therefore, in a particular embodiment, the pharmaceutical composition provided by the present invention may also comprise more than one active compound as required for the particular indication being treated, preferably those with complementary activities that do not adversely affect each other. For example, it may be desirable to further provide a chemotherapeutic agent, a cytokine, an analgesic agent, an anti-inflammatory agent or an immunosuppressive agent. The effective amount of said other active agents depends, among other things, on the therapeutic amount of aptamers or complexes that are present in the pharmaceutical composition, the nature and severity of the pathology to be treated, the subject, etc.

The aptamers or the complex of the invention may be in the composition of the invention in a therapeutically effective amount. The term "a therapeutically effective amount", in the context of the present invention, is related to the amount of the aptamer or complex of the invention that is required to achieve prevention, curing, delay, reduction in severity of, or improvement of one or more appreciable symptoms of the pathology characterised by a decrease in expression of the FPR2 receptor and/or a decrease in increased activation of the FPR2 receptor, and/or an absence/low quantity of the natural ligand of the receptor FPR2 (low quantity with respect to the quantity of natural ligand under normal health conditions).

In a particular embodiment of the composition of the invention, the aptamer of the invention or the complex of the invention are formulated with vehicles that will protect said products against rapid removal from the body, such as a controlled release formulation, including implants and microencapsulated delivery systems. Biodegradable and biocompatible polymers can be used, such as ethylene-vinyl acetate, polyanhydrides, polyglycolic acid, collagen, polyorthoesters, and polylactic acid. The methods for the preparation of such formulations will be apparent to those skilled in the art.

The pharmaceutical composition provided by the present invention can be administered to a subject by means of any suitable route of administration, such as, for example, but not limited to, parenteral route, oral route, topical route, ophthalmic route, inhalation or intranasal route.

The term "parenteral", in the context of the present invention, includes intravenous administration, intraperitoneal, intramuscular, or subcutaneous. The intravenous form of parenteral administration is generally preferred.

In addition, the pharmaceutical compositions provided by the present invention can be suitably administered by pulse infusion, for example, with decreasing doses of the aptamer or the complex of the invention. Preferably, dosing is provided by means of injections, more preferably intravenous or subcutaneous injections, partially depending on whether the administration is brief or chronic.

In another particular embodiment, the pharmaceutical compositions provided by the present invention can be adapted for parenteral administration with the addition of sterile solutions, suspensions or lyophilised products in the appropriate dosage form. Pharmaceutical compositions suitable for injectable use include sterile aqueous solutions or sterile dispersions or powders for preparing sterile injectable solutions or dispersions. For intravenous administration, suitable vehicles include physiological saline, CremophorEM bacteriostatic water (BASF, Parsippany, NJ) or phosphate buffered saline (PBS). In all cases, the composition must be sterile and fluid to facilitate injectability. It must be stable under the manufacture and storage conditions and must be preserved against the contaminating action of microorganisms such as bacteria and fungi. The vehicle can be a solvent or dispersion medium containing, for example, water, ethanol, a pharmaceutically acceptable polyol such as glycerol, propylene glycol, liquid polyethylene glycol, and suitable mixtures thereof. Suitable fluency can be maintained, for example, by using a coating such as lecithin, by maintaining the required particle size in the case of dispersion and due to the use of surfactants. Prevention of the action of microorganisms can be achieved by means of various antibacterial and antifungal agents, for example, parabens, chlorobutanol, phenol, ascorbic acid, thimerosal, and the like. In many cases, it will be preferable to include isotonic agents in the composition, for example, sugars, poly alcohols such as mannitol, sorbitol, and sodium chloride. Prolonged absorption of injectable compositions can be brought about by including an agent that delays absorption in the composition, for example, aluminium monostearate and/or gelatin.

Sterile injectable solutions can be prepared by incorporating the required amount of the active compound (for example, an aptamer or complex of the invention) in an appropriate solvent with one or a combination of the ingredients listed above, as required, followed by filter sterilisation. Generally, dispersions are prepared by incorporating the active compound into a sterile vehicle containing a basic dispersion medium and the other ingredients required from those listed above. In the case of sterile powders for the preparation of sterile injectable solutions, preferred methods of preparation are vacuum drying and freeze drying which produces a powder of the active ingredient plus any additional desired ingredients from a sterile, previously filtered solution thereof.

In a particular embodiment, said pharmaceutical composition is administered via the intravenous route. Suitable excipients can be used, such as load agents, buffering agents or surfactants. The formulations mentioned will be prepared using standard methods such as those described or envisaged in the Spanish and United States pharmacopoeias and similar reference texts.

It is especially advantageous to formulate pharmaceutical compositions, namely, parenteral compositions, in dosage unit form for ease of administration and uniformity of dosage. Dosage unit form as used herein is related to physically discrete units suitable as unit dosages for the subject to be treated; each unit containing a predetermined quantity of active compound (an aptamer or complex of the invention) calculated to produce the desired therapeutic effect in association with the required pharmaceutical vehicle. The specification for the dosage unit forms of the invention are dictated by and directly dependent on the unique features of the active compound and the particular therapeutic effect to be achieved, and the limitations inherent in the art of composing such an active compound for the treatment of subjects.

Pharmaceutical compositions containing compounds of the invention may be in a form suitable for oral use, for example, as tablets, pills, pills, aqueous or oily suspensions, dispersible powders or granules, emulsions, hard or soft capsules, or syrups or elixirs. Compositions intended for oral use may be prepared according to any method known in the art for manufacturing pharmaceutical compositions and such compositions may contain one or more agents selected from the group consisting of a sweetening agent such as sucrose, lactose or saccharin, flavourings. Agents such as mint, wintergreen or cherry oil, colourants and preservatives to provide pharmaceutically elegant and palatable preparations. The tablets containing the compounds of the invention in admixture with non-toxic pharmaceutically acceptable excipients can also be manufactured by means of known methods. The excipients used can be, for example, (1) inert diluents such as calcium carbonate, lactose, calcium phosphate or sodium phosphate; (2) granulating and disintegrating agents such as corn starch, potato starch or alginic acid; (3) binding agents such as tragacanth, corn starch, gelatin or acacia, and (4) lubricating agents such as magnesium stearate, stearate acid or talcum.

Tablets may be uncoated or may be coated by means of known techniques to delay disintegration and absorption from the gastrointestinal tract and, therefore, provide a sustained action for a longer period. For example, a time delay material such as glyceryl monostearate or glyceryl distearate may be used.

In some cases, formulations for oral use may be in the form of hard gelatin capsules in which the compounds of the invention are mixed with an inert solid diluent, for example, calcium carbonate, calcium phosphate or kaolin. They can also be in the form of soft gelatin capsules in which the compounds of the invention are mixed with water or an oily medium, for example, peanut oil, liquid paraffin or olive oil.

Pharmaceutical compositions containing compounds of the invention may be in a form suitable for topical use, for example, such as oily suspensions, as solutions or suspensions in aqueous liquids or nonaqueous liquids, or as oil-in-water or water-in-oil liquid emulsions.

For ophthalmic application, the solutions are preferably prepared using a physiological saline solution as the main vehicle. The pH of such ophthalmic solutions should preferably be kept between 4.5 and 8.0 with an appropriate buffer system, a neutral but not essential pH being preferred. Formulations may also contain conventional pharmaceutically acceptable preservatives, stabilisers and surfactants.

Preferred preservatives that can be used in the pharmaceutical compositions of the present invention include, but are not limited to, benzalkonium chloride, chlorobutanol, thimerosal, phenylmercuric acetate and phenylmercuric nitrate. A preferred surfactant is, for example, Tween 80. Likewise, various preferred carriers can be used in the ophthalmic preparations of the present invention. These vehicles include, but are not limited to, polyvinyl alcohol, povidone, hydroxypropyl methyl cellulose, poloxamers, carboxymethyl cellulose, hydroxyethylcellulose cyclodextrin and purified water.

Tonicity adjusters can be added as required or convenient. They include, but are not limited to, salts, particularly sodium chloride, potassium chloride, mannitol and glycerin, or any other suitable ophthalmically acceptable tonicity adjuster.

Various buffers and media can be used to adjust the pH as long as the resulting preparation is ophthalmically acceptable. Accordingly, buffers include acetate buffers, citrate buffers, phosphate buffers and borate buffers. Acids or bases can be used to adjust the pH of these formulations as required.

Similarly, an ophthalmically acceptable antioxidant for use in the present invention includes, but is not limited to, sodium metabisulphite, sodium thiosulphate, acetylcysteine, butylhydroxyanisole and butylate.

Other excipient components that can be included in ophthalmic preparations are chelating agents. The preferred chelating agent is disodium edodate, although other chelating agents can also be used instead or in conjunction with it. Ingredients are generally used in the following amounts: ingredient amount (% w / v), active ingredient about 0.001-5, preservative 0-0.10, vehicle 0-40, tonicity adjuster 0-10, buffer 0,01-10, pH adjuster q.s. pH 4.5-7.8, antioxidant as required, surfactant as required, purified water to make 100%.

The active compounds (aptamer or complex of the invention) will typically be administered one or more times per day, for example 1, 2, 3 or 4 times daily, with typical total daily doses in the range of 0.0001 to 1.000 mg/kg of body weight/day, preferably from about 0.001 to about 100 mg/kg of body weight/day, more preferably about 0.01 to 10 mg/kg of body weight/day. Pharmaceutical compositions can be formulated to contain the desired amount, such as a therapeutically effective amount of the aptamer or complex of the invention.

The pharmaceutical compositions provided by the present invention can be included in a recipient, container, or dispenser together with instructions for the administration thereof.

### Medical uses of the aptamer, of the complex or of the composition of the invention

The underlying cause of many diseases is due to the low activity or inhibition of a cellular receptor, which entails the locking or inhibition of the biological pathway dependent on the activation of said receptor. Therefore, a molecule that acts as an activator or agonist of a cellular receptor, and that is able to increase or activate the associated biological pathway, can be useful in the prophylaxis or treatment of a disease.

Therefore, in one aspect, the present invention is related to the aptamer of the invention, a complex of the invention or the composition of the invention for use as a medicine.

In another aspect, the present invention is related to the aptamer of the invention, a complex of the invention or the composition of the invention, for use in the prophylaxis/prevention and/or treatment of a disease, condition or pathology characterised by a decrease or inhibition of the expression of the FPR2 receptor, and/or a decrease or lock in the activation of the receptor FPR2, and/or an absence/low quantity of the natural ligand of the receptor FPR2 (low quantity with respect to the quantity of natural ligand in a subject with normal health conditions).

In the present invention, "absence" is understood as when the natural receptor ligand is in such low concentrations that its presence cannot be detected. In the present invention it is understood that an individual/subject has "normal health conditions" or is "healthy" when the individual does not have symptoms, sign or indication that he is sick or ill.

In the present invention by "treatment" or "therapy" is understood, the clinical intervention in an attempt to cure, delay, reduce the severity of, or improve one or more symptoms of the pathology characterised by a decrease or inhibition of the expression of the FPR2 receptor, and/or a decrease or lock in the activation of the receptor FPR2, and/or an absence/low quantity of the natural ligand of the receptor FPR2 (low quantity with respect to the quantity of natural ligand under normal health conditions). In the present invention, "prophylaxis" or "prevention" or "prevent" is understood as the action of preventing the development or progress of a disease or pathology such as that indicated above.

In a particular embodiment, the disease is selected from the group consisting of cancer, an autoimmune disease, an inflammatory disease, a neurodegenerative disease, a cardiovascular disease, an infectious disease, a lung disease, a vascular disease, a metabolic disease, an eye disease and an epithelial disease.

The term cancer, as used herein, is also related to tumours, proliferative diseases, malignant tumours and the metastases thereof. Examples of cancerous diseases include, but are not limited to, adenocarcinoma, choroidal melanoma, acute leukaemia, acoustic neuroma, ampullary carcinoma, anal carcinoma, astrocytoma, basal cell carcinoma, pancreatic cancer, desmoid tumour, bladder cancer, bronchial carcinoma, non-small cell lung cancer (NSCLC), breast cancer, Burkitt's lymphoma, corpus cancer, CUP syndrome (carcinoma of unknown primary origin), colorectal cancer, cancer of the small intestine, tumours of the small intestine, ovarian cancer, endometrial carcinoma, ependymoma, types of epithelial cancer, Ewing tumours, gastrointestinal tumours, gastric cancer, gallbladder cancer, gallbladder carcinomas, uterine cancer, cervical cancer, cervix, glioblastoma, gynaecological tumours, ear, nose and throat tumours, haematological neoplasms, hairy cell leukaemia, urethral cancer, skin cancer, skin testicular cancer, brain tumours (gliomas), brain metastases, testicular cancer, pituitary tumour, carcinoids, Kaposi's sarcoma, laryngeal cancer, germ cell tumours, bone cancer, colorectal carcinoma, head and neck tumours (tumours of the ear, nose and throat area), colon carcinoma, craniopharyngiomas, mouth cancer (cancer in the mouth area and on the lips), cancer of the central nervous system, liver cancer, liver metastases, leukaemia, eyelid tumour, lung cancer, lymph node cancer (Hodgkin's/Non-Hodgkin's), lymphoma, stomach cancer, malignant melanoma, malignant neoplasm, malignant tumours of the gastrointestinal tract, breast carcinoma, rectal cancer, medulloblastoma, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, oesophageal carcinoma, osteolytic carcinomas and osteoplastic carcinomas, osteosarcomas, ovarian carcinoma, pancreatic carcinoma, pancreatic carcinoma, pancreatic carcinoma, plasmacytoma, squamous cell carcinoma of head and neck (SCCHN), prostate cancer, pharyngeal cancer, rectal carcinoma, retinoblastoma, vaginal cancer, thyroid carcinoma, Schneeberger's disease, oesophageal cancer, spinaliomas, T-cell lymphoma (mycosis fungoides), thymoma, fallopian tube carcinoma, eye tumours, urethral cancer, urological tumours, urothelial carcinoma, vulvar cancer, appearance of warts, soft tissue tumours, soft tissue sarcoma, Wilms' tumour, cervical carcinoma and tongue cancer.

The aptamer of the invention, the complex of the invention or the composition of the invention, are useful for preventing and/or treating eye diseases including, inter alia, uveitis, dry eye, keratitis, allergic eye disease, infectious keratitis, herpetic keratitis, corneal angiogenesis, lymphangiogenesis, uveitis, retinitis and choroiditis, such as acute multifocal placoid pigment epitheliopathy, Behcet's disease, postoperative healing of the corneal wound, conditions caused by laser, conditions caused by photodynamic therapy, wet and dry age-related macular degeneration (ARMD), conditions affecting the rear portion of the eye, such as maculopathies and retinal degeneration, including age-related non-exudative macular degeneration, exudative age-related macular degeneration, choroidal neovascularisation, diabetic (proliferative) retinopathy, retinopathy of prematurity (ROP), acute macular neuroretinopathy; central serous retinopathy, cystoid macular oedema, and diabetic macular oedema; bird retinochoroidopathy, intermediate uveitis (pars planitis), multifocal coroiditis, multiple evanescent white dots syndrome (MEWDS), ocular sarcoidosis, posterior scleritis, serpiginous choroiditis, subretinal fibrosis syndrome and uveitis, Koyanagi and Harada syndrome; vascular diseases/exudative diseases such as arterial retinal occlusive disease, central retinal vein occlusion, cystoid macular oedema, disseminated intravascular coagulopathy, branch retinal vein occlusion (BRVO), changes in hypertension in the back of the eye, ocular ischemia syndrome, retinal arterial microaneurysms, Coats disease, juxtafoveal telangiectasis, occlusion of the hemi-retinal vein, papilliophlebitis, central retinal artery occlusion, occlusion of a branch of the retinal artery, carotid artery disease (CAD), frosted branch angiitis, sickle cell retinopathy and other haemoglobinopathies, angioid streaks, familial exudative vitreoretinopathy and Eales disease; traumatic/surgical conditions such as sympathetic ophthalmia, uveitic retinal disease, retinal detachment, trauma, conditions caused by photodynamic therapy, photocoagulation, hypoperfusion during surgery, radiation retinopathy and bone marrow transplant retinopathy; proliferative disorders such as proliferative vitreous retinopathy and epiretinal membranes, and proliferative diabetic retinopathy; infectious disorders such as ocular histoplasmosis, ocular toxocariasis, presumed ocular histoplasmosis syndrome or POHS, endophthalmitis, toxoplasmosis, retinal diseases associated with HIV infection, choroidal disease associated with HIV infection, uveitic disease associated with HIV infection, viral retinitis, acute retinal necrosis, progressive external retinal necrosis, fungal retinal diseases, ocular syphilis, ocular tuberculosis, diffuse unilateral subacute neuroretinitis and myiasis; genetic disorders such as retinitis pigmentosa, systemic disorders with associated retinal dystrophies, stationary congenital night blindness, cone dystrophies, Stargardt disease and fundus flavimaculatus, Best disease, pattern dystrophy of the retinal pigmented epithelium, X-chromosome linked retinoschisis, Sorsby's fundus dystrophy, benign concentric maculopathy, Bietti's crystalline dystrophy and pseudoxanthoma elasticum; tears/holes in the retina, like retinal detachment, macular hole and giant retinal tear; tumours such as tumour-associated retinal disease, congenital hypertrophy of the retinal pigmented epithelium, posterior uveal melanoma, choroidal haemangioma, choroidal osteoma, choroidal metastasis, combined hamartoma of the retina and retinal pigmented epithelium or CHRRPE, retinoblastoma, vasoproliferative tumours of the ocular fundus, retinal astrocytoma and intraocular lymphoid tumours; and other various diseases that affect the rear portion of the eye, such as punctate internal choroidopathy, acute posterior multifocal placoid epitheliopathy pigmentosa, myopic retinal degeneration and acute retinal pigment epitheliitis, postsurgical corneal inflammation, blepharitis, MGD, glaucoma, branch occlusion, Best vitelliform macular degeneration, retinitis pigmentosa, proliferative vitreoretinopathy (PVR) and any other degenerative disease of the photoreceptors or retinal epithelial pigment (RPE).

In the present invention, "autoimmune disease" is understood as any of a group of diseases or disorders in which tissue damage is associated with a humoural and/or cellular immune response to body constituents or, in a broader sense, a self-directed immune response. The pathological immune response can be systemic or organ specific. In other words, for example, and not limited to, self-directed immune response can affect the joints, the skin, the myelin sheath that protects neurons, kidneys, liver, pancreas, thyroid, adrenals and ovaries.

Examples of autoimmune eye diseases include, but are not limited to, idiopathic optic neuritis, sympathetic ophthalmia, anterior uveitis and other forms of uveitis, retinal degeneration and Mooren's ulcer. Examples of autoimmune skin diseases include, but are not limited to, blister pemphigoids, chronic urticaria (autoimmune subtype), dermatitis herpetiforme (Duhring's disease), epidermolysis bullosa acquisita (EBA), acquired angioedema, herpes gestationis, hypercomplementemic urticarial vasculitis (HUV), and pemphigus. Examples of haematologic autoimmune diseases include, but are not limited to, autoimmune haemolytic anaemia, autoimmune neutropenia, Evans' syndrome, inhibitory haemophilia, idiopathic thrombocytopenic purpura (ITP) and pernicious anaemia. Some examples of autoimmune heart diseases include, but are not limited to, congenital heart block, idiopathic dilatative cardiomyopathy, peripartum cardiomyopathy, postcardiotomy syndrome and post-infarction syndrome (Dressler's syndrome). Some examples of autoimmune diseases of the ear, the nose and throat include, but are not limited to, chronic sensorineural hearing loss and morbus Meniere. Examples of autoimmune diseases of the colon include, but are not limited to, autoimmune enteropathy, ulcerative colitis, indeterminate colitis, Crohn's disease and gluten-sensitive enteropathy. Examples of autoimmune disorders include, but are not limited to, autoimmune polyglandular syndrome type 1, autoimmune polyglandular syndrome type 2, type 1 diabetes mellitus (IDDM), Hashimoto-thyroiditis, insulin-autoimmune-syndrome (IAS), idiopathic diabetes insipidus, hypoparoscopic idiopathic, and Graves-Basedow disease. Some examples of autoimmune liver diseases include, but are not limited to, autoimmune hepatitis (AIH type 1, 2 and 3), primary biliary cirrhosis (PBC) and primary sclerosing cholangitis. Examples of autoimmune lung diseases include, but are not limited to, Goodpasture's syndrome. Examples of an autoimmune disease of the stomach include, but are not limited to, chronic atrophic gastritis (type A). Examples of neurological autoimmune disorders include, but are not limited to, Guillain-Barre syndrome, neuropathy associated with IgM gammopathy, Lambert-Eaton syndrome, Miller-Fisher syndrome, multiple sclerosis, multifocal motor neuropathy, myasthenia gravis, paraneoplastic neurological syndrome, Rasmussen encephalitis and stiff man syndrome. Examples of autoimmune kidney diseases include, but are not limited to, anti-TBM nephritis, Goodpasture's syndrome/anti-GBM nephritis, IgA nephropathy, interstitial nephritis and membranoproliferative glomerulonephritis. Other diseases that can be caused by an autoimmune reaction include, but are not limited to, Behcet's disease, chronic fatigue immune dysfunction syndrome (CFIDS), Cogan I syndrome, endometriosis, HELLP syndrome, Bechterew's disease, polymyalgia rheumatica, psoriasis, sarcoidosis and vitiligo.

The pathological immune response can be systemic or organ specific. In other words, for example, self-directed immune response can affect the joints, the skin, the myelin sheath that protects neurons, kidneys, liver, pancreas, thyroid, adrenals and ovaries.

In a particular embodiment, the disease is a neurodegenerative disease. Examples of neurodegenerative diseases that can be prevented and/or treated with the aptamer of the invention, the complex of the invention or the composition of the invention include, but are not limited to, Alzheimer's disease and Pick's disease, diffuse Lewy body disease, progressive supranuclear palsy (Steel-Richardson syndrome), multisystem degeneration (Shy-Drager syndrome), chronic epileptic conditions associated with neurodegeneration, motor neuron diseases (amyotrophic lateral sclerosis), multiple sclerosis, degenerative ataxias, basal cortical degeneration, ALS-Parkinson-dementia complex of Guam, subacute sclerosing panencephalitis, Huntington's disease, Parkinson's disease, synucleinopathies (including multisystem atrophy), primary progressive aphasia, striatonigral degeneration, Machado-José disease or spinocerebellar ataxia type 3 and olivopontocereferis pseudobulbar, spinal palsy and spinobulbar muscular atrophy (Kennedy's disease), primary lateral sclerosis, familial spastic paraplegia, Werdnig-Hoffmann disease, Kugelberg-Welander disease, Tay-Sach disease, Sandhoff disease, familial spastic disease, familial spastic disease, Wohlfart-Kugelberg-Welander disease, progressive parasitic spasm disease Leukoencephalopathy, familial dysautonomia (Riley-Day syndrome) or prion diseases (including, inter alia, Creutzfeld-Jakob disease, Gerstmann-Strussler-Scheinker disease, Kuru's disease or insomnia. In addition, trauma and progressive injury to the nervous system can occur in various psychiatric disorders, including, among others, progressive and impaired forms of bipolar disorder or schizoaffective disorder or schizophrenia, impulse control disorders, obsessive compulsive disorder (OCD), changes in temporal lobe epilepsy and personality disorders.

In another particular embodiment, the disease is an infectious disease. Examples of infectious diseases that can be prevented and/or treated with the aptamer of the invention, the complex of the invention or the composition of the invention include, but are not limited to, AIDS, alveolar hydatid disease (AHD, echinococcosis), amebiasis (infection by *Entamoeba histolytica*), infection by Angiostrongylus, anisakiasis, anthrax, babesiosis (Babesia infection), infection by Balantidium (balantidiasis), infection by Baylisascaris, schistosomiasis, infection by *Blastocystis hominis* (blastomycosis), borreliosis, botulism, Brainerd's diarrhoea, brucellosis, bovine spongiform encephalopathy (BSE), candidiasis, capillariasis(Capillaria infection), chronic fatigue syndrome (CFS), Chagas disease (American tranosis) (Varicella-Zoster Virus), infection by *Chlamydia pneumoniae,* cholera, Creutzfeldt-Jakob disease (CJD), clonorchiasis (infection by Clonorchis), Cutaneous larva migrans (CLM) (hookworm infection), coccidioidomycosis, conjunctivitis, hand, foot and mouth disease (infection by Coxsackie virus), cryptococcosis, infection by Cryptosporidium (cryptosporidiosis), Culex mosquito (vector of the West Nile virus), cyclosporiasis (Cyclospora infection), cysticercosis (neurocysticercosis), infection by cytomegalovirus, dengue, infection by Dipylidium (cat and dog tapeworms), infection by the Ebola virus, entamoeba fevers, and infection (amoebic dysentery), infection by Entamoeba polecki, enterobiasis (pinworm infection), infection by enterovirus (not polio), infection by Epstein-Barr virus, infection by *Escherichia coli,* foodborne infection, foot and mouth disease, fungal dermatitis, gastroenteritis, group A streptococcus disease, group B streptococcus disease, Hansen's disease (leprosy), Hantavirus pulmonary syndrome, lice infestation (pediculosis), infection by *Helicobacter pylori,* haematological disease, infection by the Hendra virus, hepatitis (HCV, HBV), herpes zoster infection, herpes zoster, human ehrlichioses, human parainfluenza virus infection, influenza, isosporiasis (Isospora infection), Lassa fever, leishmaniasis, Kala-azar (Kala-azar infection, Leishmania), lice (body lice, head lice, pubic lice), Lyme disease, malaria, Marburg haemorrhagic fever, measles, meningitis, mosquito-borne diseases, infection by *Mycobacterium avium* complex (MAC), infection by Naegleria, nosocomial infections, non-pathogenic infection of intestinal amoebae, onchocerciasis (river blindness), opisthorchiasis (Opisthorcis infection), parvovirus infection, plague, pneumocystis carinii pneumonia (PCP), polio, Q fever, rabies, respiratory syncytial virus, rheumatic fever, Rift Valley fever, river blindness (onchocerciasis), rotavirus infection, infection by pinworm, salmonellosis, salmonella enteritidis, scabies, shigellosis, shingles, sleeping sickness, smallpox, infection by streptococcus, tapeworm infection (Taenia infection), tetanus, toxic shock syndrome, tuberculosis, ulcers (peptic ulcer disease), valley fever, *Vibrio parahaemolyticus,* infection by *Vibrio vulnificus,* viral haemorrhagic fever, warts, waterborne infectious diseases, West Nile virus infection (West Nile encephalitis), whooping cough and yellow fever.

As shown in the examples in the present description, the aptamers of the invention in an *in vitro* healing assay were able to activate the migration of the human keratinocyte line HaCaT that stably expresses the FPR2 receptor. Likewise, the aptamers of the invention were also able to promote *in vivo* healing in a humanised skin animal model. Therefore, in another aspect, the present invention is related to the aptamer of the invention, a complex of the invention or the composition of the invention, for use in the treatment of epithelial or dermatological diseases, such as treating a wound.

Thus, the aptamer of the invention, the complex of the invention or the composition of the invention are useful for preventing and/or treating dermatological diseases that include, but are not limited to, rosacea, fulminant rosacea, sunburn, psoriasis, hot flashes associated with menopause, hot flashes and redness associated with hot flashes, erythema associated with hot flashes, hot flashes resulting from an orchiectomy, atopic dermatitis, treatment of redness and itching caused by insect bites, photoaging, seborrheic dermatitis, acne, allergic dermatitis, telangiectasia (dilations of previously existing small blood vessels) of the face, angioectasias, rhinophyma (hypertrophy of the nose with follicular dilation), acne-like skin rashes (oozing or scabs), burning or stinging sensation, skin erythema, skin hyperactivity with dilation of the blood vessels of the skin, Lyell's syndrome, Stevens-Johnson syndrome, local itching and discomfort associated with haemorrhoids, haemorrhoids, erythema multiforme minor, erythema multiforme major, erythema nodosum, swelling of the eyes, urticaria, itching, varicose veins, contact dermatitis, atopic dermatitis, nummular dermatitis, generalised exfoliative dermatitis, stasis dermatitis, lichen simplex chronicus, perioral dermatitis, pseudofolliculitis, granuloma annulare, actinic keratosis, basal cell carcinoma, squamous cell carcinoma, eczema, dermal wound healing, hypertrophic scars, keloid scars, melanoma, viral warts, photoaging, photodamage, melasma, postinflammatory hyperpigmentation, other pigmentation disorders, and alopecia (scarring and non-scarring forms).

In a particular embodiment, the disease is dystrophic epidermolysis bullosa (DEB). Subjects suffering from this disease, in addition to chronic wounds on the skin, show the appearance of blisters on the mucosa (mainly in the oropharynx and oesophagus), aberrant scarring leading to microstomia and oesophageal, laryngeal and nasal vestibule stricture. In addition, ophthalmologic manifestations of dystrophic epidermolysis bullosa include corneal erosions or blisters and scarring and lesions on the eyelids.

In the context of the present invention, "wound treatment" is understood as the acceleration of the wound healing process. Non-limiting examples of injuries are:
- a burn wound, which is the injury that results from exposure to heat, electricity, radiation (for example, sunburn and laser surgery), or caustic chemicals;
- grade 1, 2, 3 and 4 ulcers (classification based on the extent thereof), or pressure, venous, arterial, mixed, iatrogenic, diabetic and neoplastic/oncological ulcers (classification according to origin); mouth ulcers, corneal ulcers, genital ulcers, skin ulcers, peptic ulcers (such as gastric ulcer and duodenal ulcer, among others) (classification according to the location thereof).
- wounds in diabetes mellitus, which are usually foot injuries due to numbness caused by nerve damage (diabetic neuropathy) and decreased blood flow to the legs and feet. The most serious injury is a foot ulcer. Diabetic foot ulcers have a very high risk of being infected, and sometimes they cannot be cured. Foot ulcers that have not healed are a common cause of amputation in people with diabetes,
- pressure wounds, in other words, injuries caused by unmitigated pressure on any portion of the body, especially of portions on bone or cartilaginous areas,
- wounds due to an external force that damages the tissue,
- wounds on the skin due to aging or the environment. This includes, for example, cracks, dry skin, roughness of the skin and the like.

Wounds can be an internal wound, an oral wound, a skin wound, an external wound, an ulcer and/or a bedsore, damage to the eye, a wound in the eye, for example, conjunctiva. Other conditions that can be treated with the aptamers according to the disclosure include oral ulcers, oral aphthous lesions, glossodynia, burning tongue, psoriasis, eczema and hair loss.

These types of skin damage and lesions are known to those skilled in the art. An internal wound is a wound present in the body, for example, due to a surgical incision. An oral wound is a wound present in the oral cavity. A skin wound is a wound present on the skin. An external wound should be understood as a wound that is visible and accessible from outside the body. An ulcer is a lesion on the surface of the skin or a mucosal surface. A bedsore is a wound or ulcer caused by prolonged pressure on the skin and tissues when in one position for a long period of time, such as lying in bed. The bony areas of the body are the most frequently affected sites, which become ischemic under sustained and constant pressure. Aging of the skin is the change in the appearance of the skin due to time or exposure to the environment or the state of health of an individual. Cellulitis is the definition used in cosmetics in relation to a flaccid or wavy appearance of the skin (orange peel in Dutch).

In a particular embodiment of the invention, the aptamer, the complex or composition of the invention is used for treating a skin wound. Skin wounds can be wounds in the epidermis or dermis of the skin. There are several types of wounds wherein the skin or tissue may need repair: abrasions, lacerations, incisions, perforations and avulsions and burns. In another particular embodiment of the invention, the aptamer, the complex or composition of the invention is used for treating an oral wound. Oral wounds are wounds in any area of the oral cavity wherein the oral mucosa is damaged. In another particular embodiment of the invention, the aptamer, the complex or composition of the invention is used for treating an internal wound, in particular an injury to the internal mucosa. Internal wounds are wounds in which cell layers of endodermal or mesodermal origin are damaged. Examples include injuries to arteries or veins, peritoneum or pericardium.

In a particular embodiment, the disease is a cardiovascular disease. Examples of cardiovascular disease include, but are not limited to, aneurysm, stable angina, unstable angina, angina pectoris, angioneurotic oedema, aortic valve stenosis, aortic aneurysm, arrhythmia, arrhythmogenic dysplasia of the right ventricle, arteriosclerosis, arterial malformations, auricular fibrillation, atrial fibrillation, squad, slimming drug, slimming drug, slimming, loofah, arterial spurs, auricular fibrillation tamponade, cardiomegaly, congestive cardiomyopathy, hypertrophic cardiomyopathy, restrictive cardiomyopathy, carotid stenosis, cerebral haemorrhage, Churg-Strauss syndrome, diabetes, Ebstein anomaly, Eisenmenger complex, cholesterol embolus, bacterial endocarditis, fibromuscular, parasite, for heart failure, heart valve disease, heart attack, epidural haematoma, subdural haematoma, Hippel-Lindau disease, hyperaemia, hypertension, pulmonary hypertension, cardiac hypertrophy, left ventricular hypertrophy, right ventricular hypertrophy, hypoplastic left heart syndrome, hypotension, intermittent claudication, ischemic heart disease, Klippel-Trenaunay-Weber syndrome, lateral medullary syndrome, mitral valve prolapse of long QT syndrome, moyamoya disease, mucocutaneous lymph node syndrome, myocardial infarction, myocardial ischemia, myocarditis, pericarditis, peripheral vascular diseases, phlebitis, polyarteritis nodosa, pulmonary atresia, Raynaud's disease, Sneddon syndrome, superior vena cava syndrome, X syndrome, tachycardia, Takayasu's arteritis, hereditary haemorrhagic telangiectasia, telangiectasia, temporal arteritis, areas of paralysis of the parasite, thromboangiitis obliterans, thrombosis, tromosectos, thrombotic vasculitis, vasospasm, ventricular fibrillation, Williams syndrome, peripheral vascular disease, varicose veins and leg ulcers, deep venous thrombosis, Wolff-Parkinson-White syndrome.

Throughout the description and the claims, the word "comprises" and its variants do not intend to exclude other technical features, additives, components or steps. For those skilled in the art, other objects, advantages and features of the invention may be partially deduced from both the description and the embodiment of the invention. The following examples and figures are provided by way of illustration and are not intended to limit the present invention.

### BRIEF DESCRIPTION OF THE FIGURES

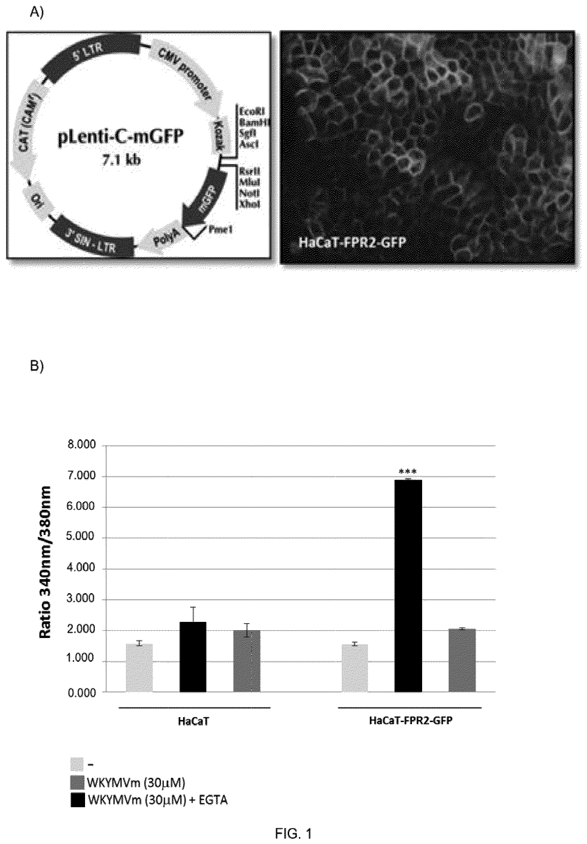

Figure 1. Generating a keratinocyte line stably expressing the FPR2 receptor A) The HaCaT keratinocyte line was stably transfected using a lentiviral vector containing an expression cassette for human FPR2 (transcript variant 1; Origene) fused with mGFP. FPR2-GFP expression on the membrane was checked by direct visualisation using an inverted microscope with epifluorescence. B) The WKYMVm peptide (FPR2 agonist) significantly induced (***p<0.001) calcium response in the HaCaT-FPR2-GFP transfectants. Cells were marked with Fura-2 in Ca2+ medium and the fluorescence intensities at 340 and 380 nm were analysed after 60 seconds of stimulation. The bar graph shows the mean ± SD of 4 replicates per condition, n=4. This effect was totally reversed after adding EGTA to the medium.

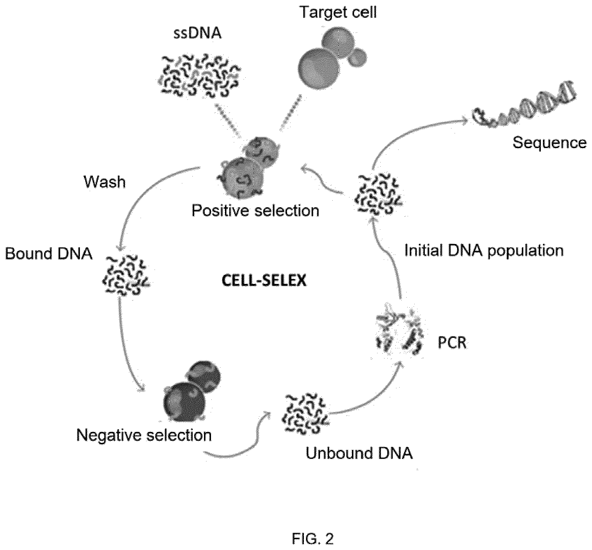

Figure 2. Obtaining aptamers for FPR2 using CELL-SELEX. The cells expressing the target protein are incubated in the presence of the aptamer population (initial RND40 population in the first cycle of SELEX or populations obtained after the different cycles). The bound aptamers are amplified by means of PCR, the complementary chain being removed, and are subjected to a new cycle of selection (positive selection) or incubated with the parental cells that do not express the target protein (negative selection). As many cycles of SELEX as necessary are carried out to obtain a population enriched in specific aptamers against the target molecule.
**Figure** 3. Study of the affinity of aptamers against FPR2. The aptamers were incubated with HaCaT-FPR2-GFP or HaCaT cells and those that bind are amplified by means of quantitative PCR (qPCR). The aptamers that bind with higher affinity are recovered in greater quantity and, therefore, is further amplified by means of PCR, obtaining a lower Ct. The ApFP3.5a, ApFP3.8a and ApFP4.5b aptamers bind with the highest affinity to FPR2.

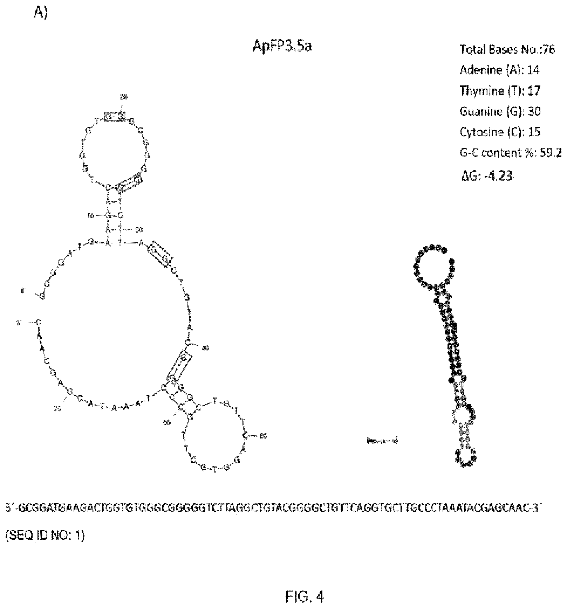

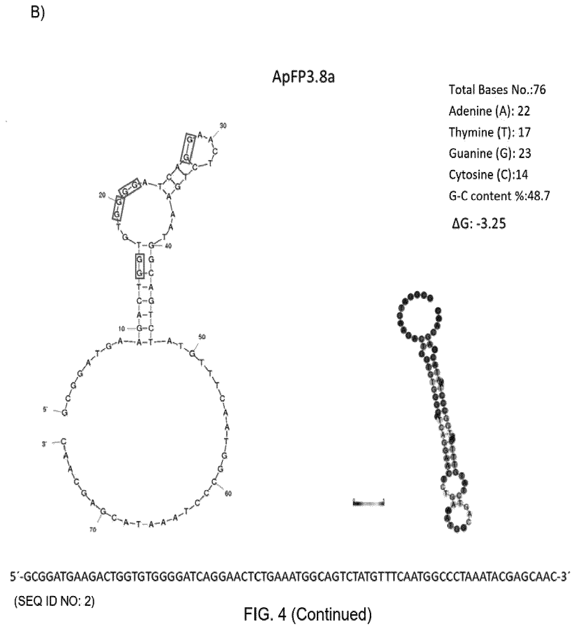

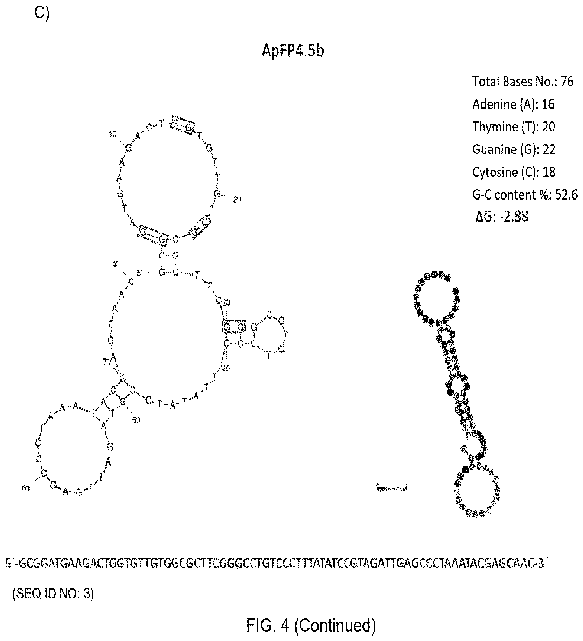

Figure 4. Secondary structures of aptamers against FPR2. A) ApFP3.5a aptamer; B) ApFP3.8a aptamer; and C) ApFP4.5b aptamer. The prediction of the secondary structures of the aptamers was carried out by means of bioinformatic analysis of the sequences thereof using the following programs: mFold (http://unafold.rna.albany.edu), RNAfold (http://rna.tbi.univie.ac.at/cgi-bin/RNAWebSuite/RNAfold.cgi) and QGRS Mapper (http://bioinformatics.ramapo.edu/QGRS/ analyze.php).

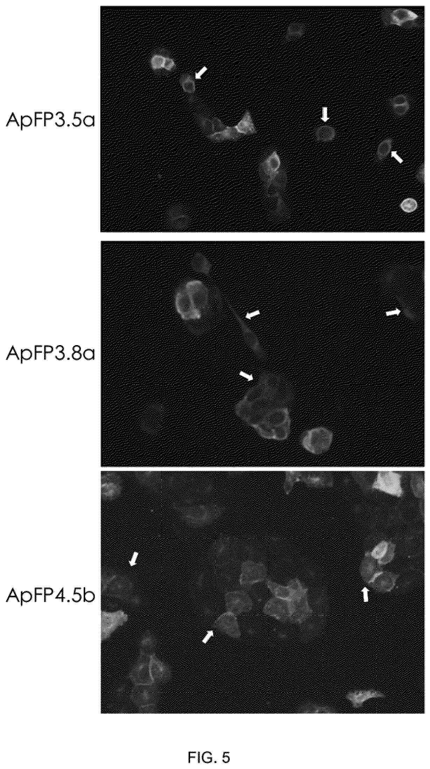

Figure 5. Colocalisation of aptamers against the FPR2 receptor (dark grey) and the protein (light grey).

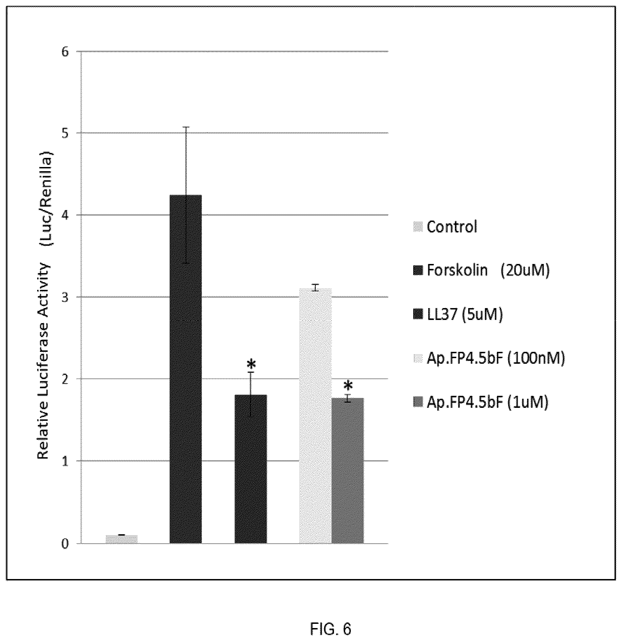

**Figure 6****. FPR2 aptamers operate as receptor agonists, inhibiting forskolin-mediated cAMP production in 293 cells**. A) 293 cells stably expressing FPR2-GFP were transiently transfected with CRE-luciferase and Renilla-luciferase reporter constructs. Twenty-four (24) hours after transfection, the indicated stimuli were added for 4 hours. The bar graph represents normalised luciferase activity versus Renilla activity for each condition, which is proportional to the amount of cAMP produced. The means were calculated using three biological replicates per condition, n=3. Error bars represent the standard deviation, S.D. A representative experiment is shown. Statistical significance (*) was determined using Student's t test (p<0.05).

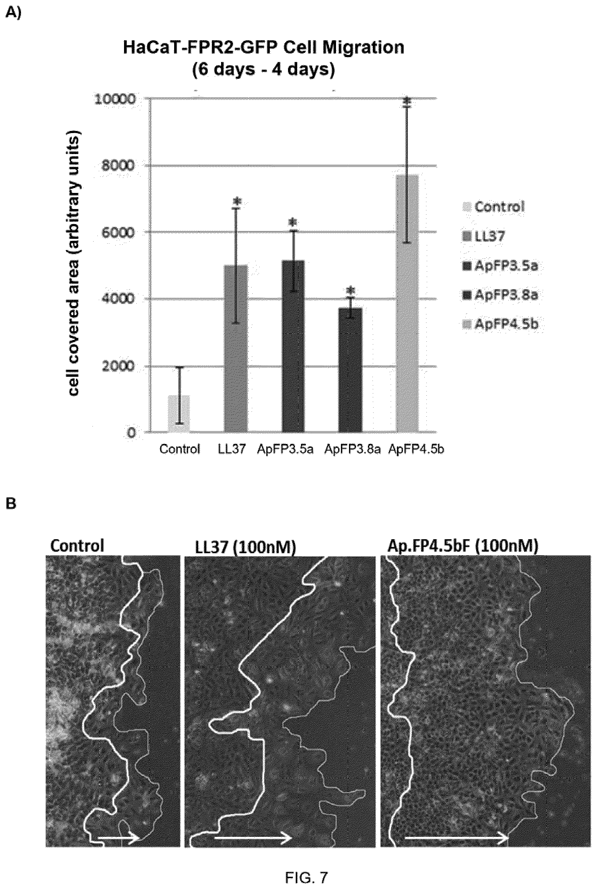

**Figure 7****. FPR2 aptamers increase keratinocyte migration in a similar manner to the LL37 agonist peptide**. The pro-healing activity of PRF2 agonists was evaluated in an *in vitro* healing assay using the stable line of HaCaT-FPR2-GFP keratinocytes. Data represent the mean ± SD of 4-6 independent fields. Statistical significance (*) was determined using Student's t test (p<0.05). The panels on the right show representative field images in each condition. The arrows show the direction of migration.

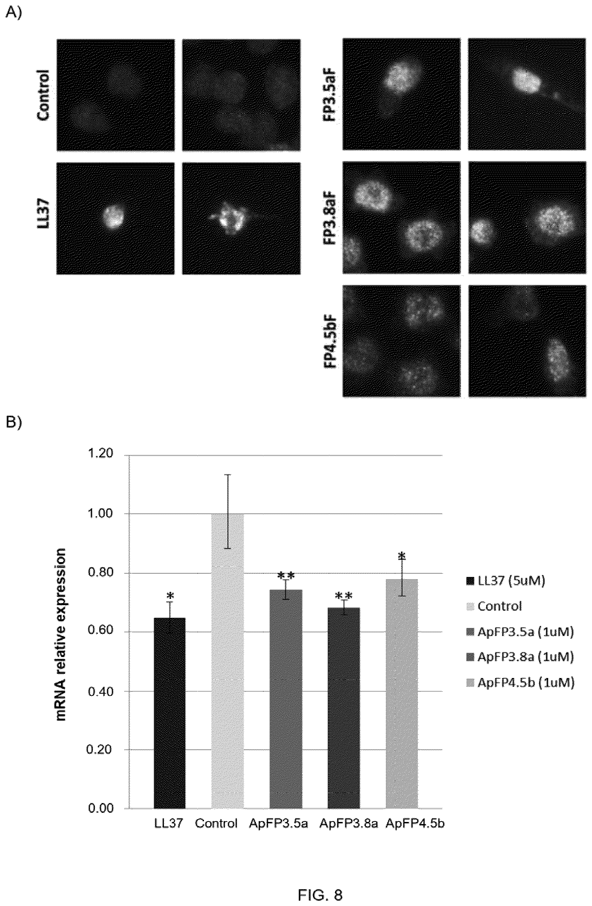

**Figure** 8. FPR2 aptamers induce CXCR2 internalisation and inhibition of its mRNA expression in THP-1 cells, in a similar manner to the LL37 agonist peptide. A) FPR2 aptamers, like the LL37 agonist peptide, are able to induce the internalisation of the CXCR2 receptor in THP-1 cells. Visualisation of the receptor was carried out by means of immunofluorescence using a specific antibody against CXCR2 in permeabilised cells. B) Aptamers against FPR2, like the LL37 agonist peptide, significantly inhibit (*p<0.05; **p<0.005) CXCR2 mRNA expression.
**Figure 9****. The FP4.5bF aptamer has prohealing activity in a humanised skin mouse model**
   A) Schematic representation of the healing test in the humanised skin mouse model. B) Histological evaluation of healing on day 6. The photographs show representative sections of H&E-stained skin tissue. In mice treated with the ApFP4.5b aptamer, a greater re-epithelialisation of the wound area (greater length of the migratory epithelial tongue) is observed in treated mice compared to control mice. C) The wounds analysed for each treatment are represented as a function of the percentage of re-epithelialisation at day 6 after the wound. Each symbol represents an individual mouse. The re-epithelialisation percentage means are represented. Error bars represent the standard deviation. Statistical significance (*) was determined using Student's t test (p<0.05).

### EXAMPLES

The invention is illustrated below by means of assays conducted by the inventors, that demonstrate the effectiveness of the product of the invention.

In the present invention, specific aptamers have been generated by means of the Cell-SELEX technique against the rhodopsin-like GPCR receptor (GPCRA) FPR2, for which cell lines have been generated that express this receptor ectopically.

### I. Materials and Methods

### Aptamer library

The inventors used the RND40 aptamer library to carry out the selection of specific aptamers against FPR2, supplied by IBA GmbH (Goettingen, Germany). The initial RND40 library is theoretically made up of 10²⁴ single-stranded DNA oligonucleotides (ssDNA) with fixed sequence at the ends, of 18 nucleotides each wherein the respective primers hybridise for amplification by means of PCR, and a central region of 40 bases of random sequence. In the selections carried out, 10¹³ oligonucleotides from this library have been used.

### Cells

The FPR2 receptor expressed in HaCaT (HaCaT-FPR2-GFP) cells is used as a target. The HaCaT human keratinocyte line, as well as line 293, were grown in DMEM culture medium with Glutamax (Gibco-BRL, Gaithersburg, MD), supplemented with 10% foetal bovine serum, at 37 °C in a 5% CO₂ atmosphere. For the generation of stable FPR2 transfectants in these cell lines, lentiviral transduction was carried out using a vector (pLenti-C-mGFP, Origene (Rockville, MD)) containing the gene encoding FPR2 fused to the green fluorescent protein GFP under the control of the CMV promoter.

### Selection with HaCaT-FPR2-GFP/HaCaT cells

For each selection round, between 8×10⁵ and 10×10⁵ HaCaT-FPR2-GFP cells were sown in triplicate in P6 plate wells, 24 hours before the selection assay and incubated at 37 °C, 5% CO2. Next, 1 nmol of aptamers from the RND40 library (or from the population isolated in the previous selection round) was added to 100 µl PBS, previously denatured at 95 °C for 10 minutes followed by incubation at 4 °C for 10 minutes, 300 µl of DMEM medium (Dulbecco's modified Eagle's medium) supplemented with 10% foetal bovine serum was added, 100 U/ml penicillin, 100 µg/ml streptomycin and 25 µg/l amphotericin and applied on the cells. After 1 hour of incubation at 37 °C, 5% CO2, the culture medium with unbound aptamers was removed, cells were washed 2 times with PBS and recovered in 500 µl of PBS by centrifugation at 1500 rpm. The cells were centrifuged to remove the supernatant and the aptamers attached to the cells were amplified by means of PCR to prepare a sufficient amount for the next selection round.

The counter-selection on HaCaT cells of the RND40 aptamer library was carried out in the previous preparation of the initial RND40 population and every 3 selection rounds, with the isolated population from the previous selection round. To that end, between 8×10⁵ and 10×10⁵ HaCaT cells were sown in triplicate in P6 plate wells, 24 hours before the selection assay and incubated at 37 °C, 5% CO2. Next, 1 nmol of aptamers from the RND40 library (or from the population isolated in the previous selection round) was added to 100 µl PBS, previously denatured at 95 °C for 10 minutes followed by incubation at 4 °C for 10 minutes, 300 µl of DMEM medium (Dulbecco's modified Eagle's medium) supplemented with 10% foetal bovine serum was added, 100 U/ml penicillin, 100 µg/ml streptomycin and 25 µg/l amphotericin and applied on the cells. After 1 hour of incubation at 37 °C, 5% CO₂, the culture medium with unbound aptamers was removed to be used in selection rounds on HaCaT-FPR2-GFP cells.

### Amplification of selected aptamers

The selected aptamers were resuspended in a volume of 20 µl of distilled water and amplified by means of PCR using the primers, which will correspond to the sequences F3 (GCGGATGAAGACTGGTGT (SEQ ID NO: 9)) and R3 (GTTGCTCGTATTTAGGGC (SEQ ID NO: 10)) under the conditions of 0.8 µM/F3 primer: 0.8 µM/R3 primer: 200 mM dNTPs, 2 mM MgCl₂, 10 U Taq polimerase (Biotools, Spain) in a final volume of 200 µl following the following amplification program: 2 minutes at 95 °C; 15 cycles of 30 seconds at 95 °C, 30 seconds at 56 °C and 30 seconds at 72 °C; and finally 5 minutes at 72 °C.

### Characterisation of selected aptamers

The selected aptamers were identified after 3 and 4 selection rounds by cloning the aptamer population in a plasmid in order to obtain individual aptamers, and subsequent Sanger sequencing.

The most represented sequences were chemically synthesised by means of IBA GmbH (Goettingen, Germany) and the affinity, the subcellular location and the activity of each of the aptamers was studied.

The nucleotide sequences of the selected aptamers are as follows:
ApFP3.5a aptamer 
GCGGATGAAGACTGGTGTGGGCGGGGGTCTTAGGCTGTACGGGGCTGTTCAGGT
GCTTGCCCTAAATACGAGCAAC (SEQ ID NO: 1)
ApFP3.8a aptamer 
GCGGATGAAGACTGGTGTGGGGATCAGGAACTCTGAAATGGCAGTCTATGTTTCA
ATGGCCCTAAATACGAGCAAC (SEQ ID NO: 2)
ApFP4.5b aptamer

GCGGATGAAGACTGGTGTTGTGGCGCTTCGGGCCTGTCCCTTTATATCCGTAGAT
TGAGCCCTAAATACGAGCAAC (SEQ ID NO: 3)

### Luciferase assay to assess cAMP production

293 cells stably expressing the FPR2-GFP receptor were sown in 96 well plates in complete medium (8.8 × 10⁵ cells per p96 plate). The next day, they were transiently transfected using Lipofectamine 3000 (Invitrogen, Carlsbad, CA) together with the pCRE-Luc reporter constructs (encoding the cAMP CRE and the Luc gene for the response element, encoding firefly luciferase), and pSV40-RL (containing an SV40 promoter and the gene encoding Renilla luciferase). Twenty-four (24) hours after transfection, cells were well stimulated with forskolin (20µM), an activator of adenylate cyclase, either with the LL37 peptide (5 µM)+ forskolin (20 µM) or with two concentrations of the ApFP4.5b aptamer (100 µM or 1 µM) + forskolin (20 µM) for 4 hours. For quantifying the luminescent signal, the Dual-Glo^{®} Luciferase Assay System kit (Promega, Madison, WI) was used, following the indications of the producer. The Luc measurements were normalised against the Renilla measurements.

### In vitro migration tests

Cells were grown to confluence in 6-well culture plates. At that time the cells were serum starved for 24 hours. The aptamers and the LL37 peptide were tested at concentrations of 100 nM in an *in vitro* healing assay, where they are added after removing the cells that cover half of the surface of each well. To assess the migration, photographs were taken at different times (t = 0 hours - 2 days -4 days - 6 days) after the generation of the wound. Photographs taken on days 4 and 6 after injury were overlaid using Adobe Photoshop software (Adobe Systems, Berkeley, CA). Migration areas were measured using Image J software (NIH Image, Bethesda, MD).

### CXCR2 Internalisation Assays

THP-1 cells grown on coverslips were treated with the LL37 peptide (5 uM) and with the FPR2 aptamers (5 µM), and the CXCR2 expression was analysed by immunofluorescence after 5 hours of treatment. Cells were washed with PBS1x and fixed with 2% paraformaldehyde for 15 minutes. Subsequently, the cells were permeabilised by incubating with 0.01% saponin for 30 minutes, and non-specific binding sites were locked by incubation with 10% horse serum and 0.01% saponin diluted in PBS1x for 30 minutes. Cells were incubated with a polyclonal antibody generated against human CXCR2 in rabbit (Abcam) for 1 hour at 4 °C. After washing, cells were incubated with a secondary antibody against Alexa Fluor 488-conjugated rabbit (Molecular Probes) for 30 minutes. After washing, the samples were mounted for viewing using an Axioplan 2 fluorescence microscope (Zeiss, Jena, Germany).

### Inhibition of CXCR2 mRNA expression by qPCR

The total RNA of the THP-1 cells treated or not with the LL37 (5µM) peptide or with the specific FPR2 (1 µM) aptamers was extracted using the miRNeasy Mini Kit (Qiagen, Hilden, Germany). The cDNA generation was obtained using the "high capacity reverse transcription system" kit (Applied Biosystems, Foster City, CA). The products of the RT reaction were used for their amplification by means of quantitative PCR, using specific oligonucleotides, the Power Sybr Green PCR master mix (Applied Biosystems) and the following amplification conditions: 10 minutes at 95 °C, followed by 40 cycles of 15 seconds at 95 °C and 1 minute at 60 °C. The Tata binding protein (TBP) and Ubiquitin C (UBC) reference genes were used for the normalisation procedures. Simultaneous triplicate reactions were carried out for both the target genes and the reference genes for each template cDNA analysed. The 2-ΔCT method was used to calculate the relative expression of each target gene (Livak KJ, Schmittgen TD. Analysis of relative gene expression data using real-time quantitative PCR and the 2(-Delta Delta C(T)) Method. Methods. 2001 Dec;25(4):402-8.)

### In vivo healing tests

Healing experiments were carried out on the humanised skin mouse model as previously described (Escamez et al., 2004, J invest Dermatol. 123(6): 1182-91). For this, NMRI nu (Rj: NMRI-Foxn1^{nu/nu}) immunodeficient mice 6 to 8 weeks old were used, that were orthotopically transplanted with equivalents of bioengineered human skin consisting of a fibrin matrix containing fibroblasts as a dermal component and keratinocytes as part of the epidermal component (Del Rio M, et al. 2002. Hum Gene Ther., 13(8):959-68). 10-12 weeks after transplantation, excisional wounds were made in the regenerated human skin using 2 mm biopsy punches. The administration of the treatments was carried out through the implantation of osmotic pumps (Alzet, Cupertino, CA) subcutaneously. The ApFP4.5b aptamer dose was 10 µM in all mice. The tissues were collected 6 days after wound generation, they were fixed in formalin and processed for subsequent histological analysis. The samples were fully sectioned using a microtome and the tissue architecture was subsequently determined in 1 out of 10 sections by means of haematoxylin-eosin staining, following standard histological methods. The re-epithelialisation percentage was determined by means of microscopy using a grating to measure the proportion of each wound that had been covered by neoepidermis in relation to the total length of the wound. Thus, this percentage was calculated by the formula 100x [(wound diameter-epidermal gap)/wound diameter]. The epidermal gap is the distance between the two epithelial tongues. Thus, the centre of the wound was determined in each sample, and the different re-epithelialisation percentages between the different samples were compared.

### II. Results

Assays *for binding the aptamers to FPR2 expressed in cells.*

In order to analyse the ability of the identified aptamers to bind to the FPR2 protein expressed in HaCaT cells, 20 pmol of each of the aptamers identified above were added to a culture of HaCaT-FPR2-GFP cells sown at 20,000 cells/well in 96-well microtiter plates at a density of 2×10⁴ cell/well, 2 days before the start of the assay. After incubation for 30 minutes at 37 °C, 5% CO2, the cells were washed, aptamers were recovered with 150 mM imidazole dissolved in PBS with 1 mM MgCl₂, and qPCR was carried out to determine Ct values. In these experiments, a lower Ct value indicates a greater amount of aptamer bound to the cells. The results obtained show that the ApFP3.5a, ApFP3.8a and ApFP4.5b aptamers (Figure 3) are the ones that bind with the highest affinity, the usefulness thereof in the detection of the protein FPR2 being demonstrated. The sequences and secondary structures of these aptamers are shown in Figure 4.

*Subcellular localisation by means of aptacytochemistry.*

Cells expressing the FPR2 receptor fused to green fluorescent protein were incubated in the presence of aptamers marked with AlexaFluor 700 ApFP3.5a, ApFP3.8a and ApFP4.5b in selection buffer for 1 hour at room temperature. Subsequently, cells were washed three times with PBS. Lastly, cells were mounted on glass slides using a glycerol buffer containing p-phenylenediamine and a 1/750 dilution of Dapi for nuclear staining. Controls were made by omitting the aptamer. The subcellular location of the aptamers was evaluated by means of fluorescence microscopy. The results show that there is a colocalisation of the aptamers with the protein (Figure 5).

*Functionality of the selected aptamers against FPR2.*

In these assays, different approaches were used. First, a typical GPCR assay (CRE-luciferase reporter activity assay) was carried out, using a stable transfectant of the FPR2 receptor generated in the 293 cell line by means of lentiviral transduction (Figure 6). This line was transiently transfected with two reporter constructs (CRE-luciferase and Renilla-luciferase). The LL37 peptide, FPR2 receptor agonist, enables the Gi subunit receptor coupling resulting in the inhibition of forskolin-mediated cAMP production. FPR2-specific aptamers are also able to inhibit forskolin-mediated cAMP production in a manner similar to LL37 (Table 1). These results reveal the ability of the identified aptamers to activate the FPR2 receptor.

**Table 1. The table indicates the percentages of inhibition of forskolin-mediated cAMP production. Values indicate the mean ± SEM of the data obtained from 3-6 independent experiments.**

| | Inhibition (%) of forskolin-mediated cAMP production |
|---|---|
| LL37 (5µM) | 46 ± 7.5 |
| Ap.FP3.5aF (1µM) | 30.7 ± 7.3 |
| Ap.FP3.8aF (1µM) | 38.8 ± 9 |
| Ap.FP4.5bF (1µM) | 25.5 ± 2.8 |

*Utility of selected aptamers in healing.*

In an *in vitro* healing test the three specific aptamers against FPR2 are able to activate the migration of the HaCaT human keratinocyte line stably expressing FPR2, as well as the LL37 peptide (Figure 7). Specific FPR2 aptamers are also able to act as functional CXCR2 ligands, since they induce receptor internalisation in the THP-1 human monocyte line, as well as the inhibition of CXCR2 mRNA expression in this line, in a similar manner to what occurs when the LL37 peptide is used as an agonist (Figure 8). Likewise, *in vivo* assays were carried out with the ApFP4.5b aptamer, that was able to exert pro-healing actions, inducing an improvement in the re-epithelialisation process. The tests were carried out using a humanised skin mouse model, wherein excisional wounds were generated. Treatments were administered subcutaneously using osmotic pumps (Figure 9). Figure 9 shows how in mice treated with the ApFP4.5b aptamer a greater re-epithelialisation of the wound area is observed (greater length of the migratory epithelial tongue) in treated mice compared to control mice, demonstrating the effect of the aptamer on healing and demonstrating the usefulness of the aptamer in the treatment of epithelial wounds.

## Claims

1. A nucleic acid aptamer which is able to specifically bind to the FPR2 receptor and activate said FPR2 receptor, comprising a nucleotide sequence with a sequence identity of at least 70% with the sequence SEQ ID NO: 1, SEQ ID NO: 2 or SEQ ID NO: 3.

2. Aptamer according to claim 1, wherein the nucleotide sequence has a sequence identity of, at least 80, 90, 95, 96, 97, 98 or 99% with the sequence SEQ ID NO: 1, SEQ ID NO: 2 or SEQ ID NO: 3.

3. Aptamer according to claim 1 or 2, wherein the nucleotide sequence has a sequence identity of 100% with the sequence SEQ ID NO: 1, SEQ ID NO: 2 or SEQ ID NO: 3.

4. Aptamer according to any one of claims 1 to 3, wherein the FPR2 receptor is the human FPR2 receptor.

5. Aptamer according to any one of claims 1 to 4, wherein the nucleic acid is DNA.

6. A complex comprising an aptamer according to any one of claims 1 to 5 and a functional group.

7. Complex according to claim 6, wherein the functional group is a detectable reagent, a drug or a nanoparticle.

8. A pharmaceutical composition comprising an aptamer according to any one of claims 1 to 5, and/or a complex according to claim 6 or 7, together with a pharmaceutically acceptable carrier, excipient or vehicle.

9. *In vitro* use of an aptamer according to any one of claims 1 to 5, or a complex according to claim 6 or 7, to detect the FPR2 receptor.

10. Use according to claim 9, wherein the detection of the FPR2 receptor is carried out by means of a method selected from the group consisting of ELONA, aptacytochemistry, aptahistochemistry and flow cytometry.

11. *In vitro* use of an aptamer according to any one of claims 1 to 5, or a complex according to claim 6 or 7, to activate the FPR2 receptor.

12. *In vitro* method for the detection of the FPR2 receptor in an isolated biological sample from a subject comprising
(a) contacting said sample with an aptamer according to any one of claims 1 to 5, or a complex according to claim 6 or 7,
(b) separating the aptamer or complex which is not bound to the FPR2 receptor, and
(c) detecting the presence of the aptamer or complex which is bound to the FPR2 receptor present in the sample.

13. The method according to claim 12, wherein the detection is carried out by means of fluorescence.

14. *In vitro* method to activate the FPR2 receptor in an isolated biological sample from a subject comprising contacting said sample comprising the FPR2 receptor with an aptamer according to any one of claims 1 to 5, or a complex according to claim 6 or 7, under suitable conditions to activate the FPR2 receptor.

15. Method according to any one of claims 12 to 14, wherein the subject is a human.

16. Method according to any one of claims 12 to 15, wherein the isolated biological sample is blood, plasma, serum or cerebrospinal fluid.

17. An aptamer according to any one of claims 1 to 5, or a complex according to claim 6 or 7, or a pharmaceutical composition according to claim 8, for use in treating a wound.

18. An aptamer according to any one of claims 1 to 5, or a complex according to claim 6 or 7, or a pharmaceutical composition according to claim 8, for use in the prevention and/or treatment of diseases **characterised by** a decrease in the expression of the FPR2 receptor, and/or a decrease in the activation of the FPR2 receptor and/or an absence of the natural ligand of the FPR2 receptor and/or a low amount of the natural ligand of the FPR2 receptor with regard to the amount of natural ligand in a subject under normal health conditions or healthy.

19. Aptamer, complex or composition for use according to claim 18, wherein the disease is selected from the group consisting of cancer, an autoimmune disease, an inflammatory disease, a neurodegenerative disease, a cardiovascular disease, an infectious disease, an eye disease and an epithelial disease.

20. Aptamer, complex or composition for use according to claim 19, wherein the epithelial disease is dystrophic epidermolysis bullosa.
